# EUROPEAN PATENT SPECIFICATION

(11) **EP 1 389 212 B1**
(45) Date of publication and mention of the grant of the patent: **12.07.2006**
(21) Application number: 02723011.9
(22) Date of filing: 12.04.2002
(51) Int. Cl.: C07D 498/08, A61K 31/5386, A61P 9/06

(54) **3,7-DIAZABICYCLO 3.3.1| FORMULATIONS AS ANTI-ARRYTHMIC COMPOUNDS**
3,7-DIAZABICYCLOç3.3.1]-FORMULIERUNGEN ALS ANTIARRHYTHMIKA
PREPARATIONS DE 3,7-DIAZABICYCLO 3.3.1| EMPLOYEES COMME COMPOSES ANTI-ARYTHMISANTS

(30) Priority: 12.04.2001 SE 0101329
(43) Date of publication of application: 18.02.2004
(73) Proprietor: AstraZeneca AB, 151 85 Södertälje (SE)
(72) Inventor: HOVDAL, Christina, S-431 83 Mölndal (SE); LUNDGREN, Anna, S-431 83 Mölndal (SE)
(86) International application number: PCT/SE2002/000726
(87) International publication number: WO 2002/083689

(56) References cited:
- EP-A2- 0 308 843
- WO-A1-00/61569
- WO-A1-91/07405
- WO-A1-99/31100
- WO-A2-01/28992
- PATENT ABSTRACTS OF JAPAN & JP 07 252 152 A (KALI CHEM PHARMA GMBH) 03 October 1995

## Description

This invention relates to novel pharmaceutical formulations that provide for delivery of particular drugs, which drugs are useful in the treatment of cardiac arrhythmias.

### Background and Prior Art

It is often desirable to formulate pharmaceutically-active compounds for immediate release following oral and/or parenteral administration with a view to providing a sufficient concentration of drug in plasma within the time-frame required to give rise to a desired therapeutic response.

Immediate release may be particularly desirable in cases where, for example, a rapid therapeutic response is required (e.g. in the treatment of acute problems), or, in the case of parenteral administration, when peroral delivery to the gastrointestinal tract is incapable of providing sufficient systemic uptake within the required time-frame.

In the case of the treatment or prophylaxis of cardiac arrhythmias, immediate release formulations may be necessary to ensure that a sufficient amount of drug is provided in plasma within a relatively short period of time to enable, for example, rapid conversion of atrial fibrillation (AF) to sinus rhythm, or prevention of relapse into AF in vulnerable patients.

Immediate release formulations are also typically simpler to develop than modified release formulations, and may also provide more flexibility in relation to the variation of doses that are to be administered to patients.

International patent application WO 01/28992 discloses a number of oxabispidine compounds, including:
(a) 4-({3-[7-(3,3-dimethyl-2-oxobutyl)-9-oxa-3,7-diazabicyclo[3.3.1]non-3-yl]propyl} amino)benzonitrile: 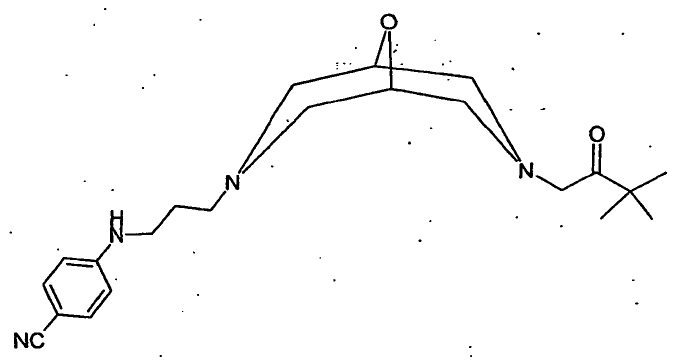 which compound is referred to hereinafter as Compound A. Compound A is specifically disclosed in WO 01/28992, both in the form of the free base and in the form of a benzenesulphonate salt;
(b) *tert*-butyl 2-{7-[3-(4-cyanoanilino)propyl]-9-oxa-3,7-diazabicyclo-[3.3.1]non-3-yl}ethylcarbamate: 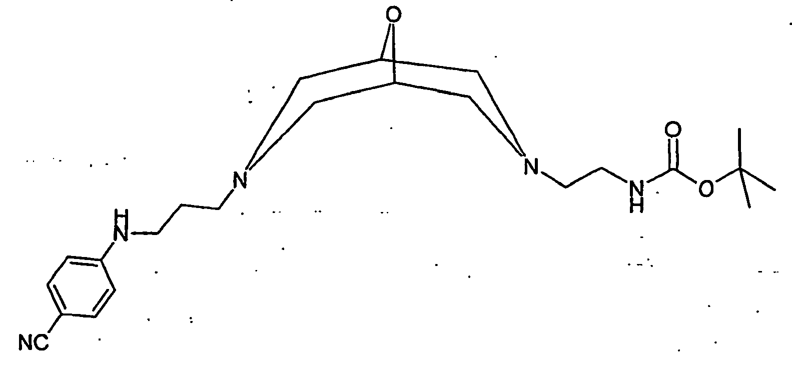 in the form of the free base, which compound is referred to hereinafter as Compound B;
(c) *tert*-butyl 2-{7-[4-(4-cyanophenyl)butyl]-9-oxa-3,7-diazabicyclo-[3.3.1]non-3-yl}ethylcarbamate: 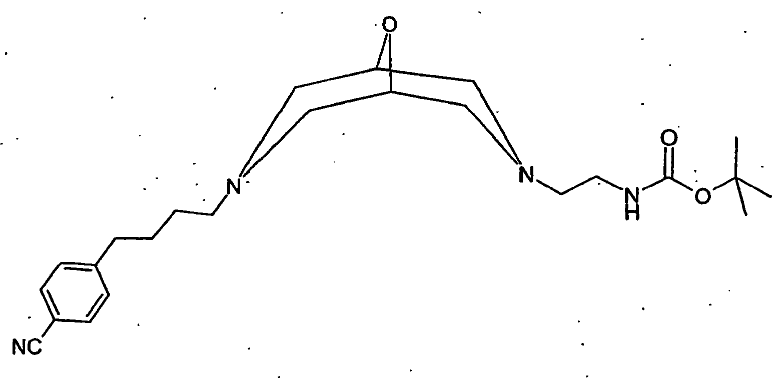 in the form of the free base, which compound is referred to hereinafter as Compound C; and
(d) *tert*-butyl 2-{7-[(2S)-3-(4-cyanophenoxy)-2-hydroxypropyl]-9-oxa-3,7-diazabicyclo[3.3.1]non-3-yl}ethylcarbamate: 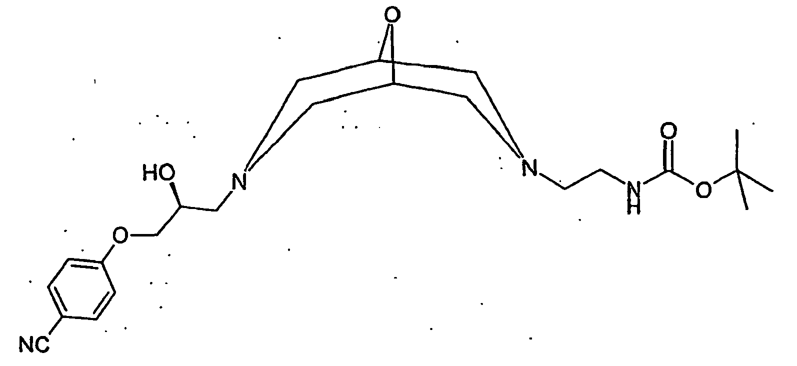 in the form of the free base, which compound is referred to hereinafter as Compound D.

The compounds of international patent application WO 01/28992 are indicated as being useful in the treatment of cardiac arrhythmias.

Although general information is provided in WO 01/28992 in relation to how the compounds disclosed therein may be formulated and thereafter administered to patients, no mention is made of immediate release pharmaceutical formulations including, specifically, Compound A, B, C or D, nor salts of any of these compounds.

We have found that it may be advantageous to provide Compound A, Compound B, Compound C and Compound D, and pharmaceutically-acceptable salts thereof, in an immediate release dosage form.

We have further found that Compounds A, B, C and D, and salts thereof, may be readily formulated as pharmaceutical formulations, as described hereinafter, which may be stable during storage and easy to administer, e.g. for oral and parenteral administration.

### Disclosure of the Invention

According to a first aspect of the invention, there is provided an immediate-release pharmaceutical formulation comprising:
(a) as active ingredient, Compound A, Compound B, Compound C, or Compound D, or a pharmaceutically-acceptable salt of any of these compounds; and
(b) a pharmaceutically-acceptable diluent or carrier,
which formulations are referred to hereinafter as "the formulations of the invention".

The term "immediate release" pharmaceutical formulation will be well understood by the skilled person to include any formulation in which the onset and/or rate of release, and/or absorption, of drug, is neither appreciably, nor intentionally, retarded by galenic manipulations. In the present case, immediate release may be provided for by way of an appropriate pharmaceutically-acceptable diluent or carrier, which diluent or carrier does not prolong, to an appreciable extent, the onset and/or rate of drug release/absorption. Thus, the term will be understood by those skilled in the art to exclude formulations which are adapted to provide for "modified" or "controlled" release, including a "sustained", "prolonged", "extended" or "delayed" release of drug.

In this context, the term "release" may be understood to include provision (or presentation) of drug from the formulation to the gastrointestinal tract, to body tissues and/or into systemic circulation.

Thus, formulations of the invention may release at least 70% (e.g. 80%) of active ingredient within 4 hours, such as within 3 hours, preferably 2 hours, more preferably within 1.5 hours, and especially within an hour (such as within 30 minutes), of administration, whether this be oral or parenteral.

The formulations of the invention may be formulated in accordance with a variety of techniques known to those skilled in the art, for example as described by M. E. Aulton in "*Pharmaceutics: The Science of Dosage Form Design"* (1988) (Churchill Livingstone).

Formulations of the invention may be, or may be adapted in accordance with standard techniques to be, suitable for peroral administration, for example in the form of immediate release tablets or capsules, or as liquid dosage forms, comprising active ingredient, both of which formulation types are well known to the skilled person and may be prepared in accordance with techniques known in the art (see below).

Suitable diluents/carriers (which may also be termed "fillers") for use in peroral formulations of the invention, for example those in the form of immediate release tablets, include monobasic calcium phosphate, dibasic calcium phosphate (including dibasic calcium phosphate dihydrate and dibasic calcium phosphate anhydrate), tribasic calcium phosphate, lactose, microcrystalline cellulose, silicified microcrystalline cellulose, mannitol, sorbitol, starch (maize, potato, rice), glucose, calcium lactate, calcium carbonate and the like. Preferred diluents/carriers include dibasic calcium phosphate and microcrystalline cellulose.

Formulations of the invention in the form of for example immediate release tablets may further comprise one or more additional excipients known to those skilled in the art for use in such formulations, to improve the physical and/or chemical properties of the final composition, and/or to facilitate the process of manufacture. Such excipients are conventional in the formulation of immediate release formulations for peroral drug delivery, and include one or more of the following: one or more of the following lubricants: magnesium stearate, stearic acid, calcium stearate, stearyl alcohol or, preferably, sodium stearyl fumarate and the like; glidants, such as talc or a colloidal silica; one or more of the following binders: polyvinylpyrrolidone, microcrystalline cellulose, a polyethylene glycol (PEG), a polyethylene oxide, a hydroxypropylmethylcellulose (HPMC) of a low molecular weight, a methylcellulose (MC) of a low molecular weight, a hydroxypropylcellulose (HPC) of a low molecular weight, a hydroxyethylcellulose (HEC) of a low molecular weight, starch (maize, potato, rice), a sodium carboxymethyl cellulose of a low molecular weight and the like (preferably polyvinylpyrrolidone or a HPMC of a low molecular weight); one or more of the following pH controlling agents: organic acids (e.g. citric acid and the like) or alkali metal (e.g. sodium) salts thereof, oxides of magnesium, as well as alkali, and alkaline earth, metal (e.g. sodium, calcium, potassium and the like) sulphates, metabisulphates, propionates and sorbates; one more of the following disintegrants: sodium starch glycolate, crosslinked polyvinylpyrrolidone, crosslinked sodium carboxymethyl cellulose, starch (maize, potato, rice), alginates and the like; as well as further excipients, including colourants, flavourings, tonicity-modifying agents, coating agents, preservatives, etc.

Combinations of the above-stated further excipients may be employed. Further, it will be appreciated by the skilled person that some of the above mentioned further excipients, which may be present in a final immediate release oral (e.g. tablet) formulation of the invention, may have more than one of the above-stated functions.

Alternatively, formulations of the invention may be, or may preferably be adapted in accordance with standard techniques to be, suitable for parenteral administration. The term "parenteral" will be understood by those skilled in the art to include any mode of administration that does not comprise peroral administration to the gastrointestinal tract. The term may thus be understood to include administration subcutaneously, intravenously, intraarterially, transdermally, intranasally, intrabuccally, intracutaneously, intramuscularly, intralipomateously, intraperitoneally, rectally, sublingually, topically, by inhalation, or by any other parenteral route.

Suitable formulations of the invention that are to be administered parenterally include those in which Compound A, Compound B, Compound C, Compound D, or pharmaceutically-acceptable salts of Compounds A, B, C and D, are presented together with an aqueous carrier, such as water.

Formulations of the invention comprising aqueous carriers may further comprise one or more additional excipients known to those skilled in the art for use in aqueous parenteral formulations, such as antimicrobial preservatives; tonicity modifiers (such as sodium chloride, mannitol, glucose and the like); pH adjusting agents (such as common inorganic acids and bases, including hydrochloric acid, sodium hydroxide and the like); pH controlling agents (i.e. buffers, such as tartaric acid, acetic acid, citric acid and the like); surfactants (e.g. Solutol^{TM}); cosolvents, which may serve to further solubilise active ingredient (such as ethanol, polyethylene glycols, hydroxypropyl-β-cyclodextrin and the like); and/or antioxidants.

The amount of further excipients that may be employed in the peroral and parenteral formulations of the invention depends upon many factors, including the nature and amount of active ingredient that is included, and the amount of diluent/carrier (aqueous solvent or otherwise) that is included, but may be in line with those amounts that are typically employed in immediate release pharmaceutical formulations that are known in the art, and/or may be determined routinely by the skilled person.

Formulations of the invention that may be, for example, suitable for parenteral administration, may be provided in the form of suspensions of active ingredient in association with an aqueous solvent or, more preferably, and especially when the formulation is to be administered parenterally (particularly intravenously), aqueous solutions (i.e. solutions of active compound including water as a solvent). In this context, the term "aqueous solution" may be understood to include formulations in which at least 99% of active ingredient is in solution at above 5°C and atmospheric pressure, and the term "suspension" should be construed accordingly (i.e. more than 1% of active ingredient is not in solution under such conditions).

Formulations of the invention that may be mentioned include those in which, when the formulation comprises Compound A, Compound A, *para-*toluenesulphonic acid salt, Compound A, benzenesulphonic acid salt, and an aqueous carrier, which formulation comprises either no additional excipients as hereinbefore defined, or ethanol as the sole additional excipient, then the formulation is in the form of a suspension of active ingredient in the carrier as hereinbefore defined.

Formulations of the invention that may be mentioned include those in which, when the formulation comprises Compound A, or Compound A, benzenesulphonic acid salt, an aqueous carrier, and ethanol as sole additional excipient, then the ethanol content is no more than 10% (w/w) of the content of the carrier.

Formulations of the invention that may be mentioned include those in which, when the formulation comprises Compound A, benzenesulphonic acid salt and an aqueous carrier, then the water content of that carrier is at least 90% (w/w).

When active ingredient is provided in the form of a salt, preferred salts are acid addition salts. Suitable inorganic acids that may be employed in the preparation of such salts include hydrochloric acid, hydrobromic acid, sulphuric acid and phosphoric acid. Suitable organic acids that may be employed include lower alkyl sulphonic acids (such as benzenesulphonic acid, a hydroxybenzenesulphonic acid, a toluenesulphonic acid, a naphthalenesulphonic acid, a naphthalenedisulphonic acid, a mesitylenesulphonic acid, methanesulphonic acid, ethanesulphonic acid and 2-hydroxyethane sulphonic acid), carboxylic acids (such as aspartic acid, maleic acid, succinic acid, malonic acid, acetic acid, fumaric acid, benzoic acid, terephthalic acid, hippuric acid, 1-hydroxy-2-naphthoic acid, pamoic acid and hydroxybenzoic acid), hydroxy acids (such as salicylic acid, glycolic acid, malic acid, ascorbic acid, citric acid, gluconic acid, lactic acid and tartaric acid) and amino acids (such as L-lysine, or L-lysine hydrochloride, such as monohydrochloride). Mono as well as polyvalent acids may be used.

Preferred acid addition salts of Compound A may be formed from 1-hydroxy-2-naphthoic acid, benzoic acid, a hydroxybenzenesulphonic (e.g. *para-*hydroxybenzenesulphonic) acid, benzenesulphonic acid, a toluenesulphonic (e.g. *para*-toluenesulphonic) acid, a naphthalenesulphonic (e.g. 1,5-naphthalenesulphonic) acid, a naphthalenedisulphonic (e.g. a 1,5-naphthalenedisulphonic) acid and a mesitylenesulphonic (e.g. 2-mesitylenesulphonic) acid, as well as from methanesulphonic acid, tartaric acid, succinic acid, citric acid, acetic acid, hippuric acid, and hydrohalic acids, such as hydrochloric acid and hydrobromic acid.

Preferred acid addition salts of Compound C may be formed from toluenesulphonic acid, methanesulphonic acid, tartaric acid, succinic acid, citric acid, acetic acid, hippuric acid, and hydrohalic acids, such as hydrochloric acid and hydrobromic acid.

Preferred acid addition salts of Compound D may be formed from L-lysine monohydrochloride, pamoic acid and terephthalic acid, as well as from methanesulphonic acid, tartaric acid, succinic acid, citric acid, acetic acid, hippuric acid, and hydrohalic acids, such as hydrochloric acid and hydrobromic acid.

Thus, the skilled person will appreciate that some of the pH controlling agents (buffers) and pH adjusting agents mentioned hereinbefore may also be employed to provide salts of Compounds A, B, C and D.

Preferred formulations of the invention include those in which active ingredient is water soluble.

By "water soluble", we include that the solubility of active ingredient in aqueous solutions, for example water, with or without the presence of any of the additional excipients mentioned hereinbefore, as measured by standard techniques, is at least 1 mg/mL, preferably at least 2 mg/mL, more preferably at least 5 mg/mL and especially at least 10 mg/mL.

Thus, pharmaceutically-acceptable salts of Compounds A, B, C and D which may be particularly mentioned include those of inorganic and organic acids, which acids may form water-soluble salts when added to Compounds A, B, C or D. It will be appreciated by the skilled person that water solubility of salts that may be formed between acids and Compound A, Compound B, Compound C and Compound D may depend upon the physical and chemical properties of the acid, including *inter alia* the water solubility (of the acid itself), lipophilicity of the counter-ion and the acid's dissociation constant.

Compound A, Compound B, Compound C, Compound D, and pharmaceutically-acceptable salts of Compounds A, B, C and D, may be made by way of routine techniques in organic chemistry, by analogy with techniques described in WO 01/28992 and/or as described hereinafter.

Formulations of the invention, such as parenteral formulations, comprising salts may be prepared by addition of diluent/carrier to the appropriate pre-prepared salt. Salts may be prepared by way of a process which involves the addition of an appropriate acid to the appropriate base (Compound A, Compound B, Compound C or Compound D). Acid and base may be reacted together in this way for example by providing either of the acid or the base in the form of a solution in an appropriate solvent, or by adding acid directly to base (which base is optionally in solution), followed by isolation of the salt or (if appropriate) addition of an appropriate solvent to provide a solution, or suspension, of salt (if and as required). In the formation of acid addition salts, suitable solvent systems may be heterogeneous or homogeneous and may thus comprise one or more organic solvents, such as alkyl acetates (e.g. linear or branched C₁₋₆ alkyl acetates, such as ethyl acetate, *iso*-propyl acetate and butyl acetate), lower (e.g. linear or branched C₁₋₆) alkyl alcohols (e.g. C₁₋₄ alkyl alcohols, such as methanol, ethanol, *iso*-propanol and butanol), chlorinated solvents (e.g. dichloromethane), ethers (such as diethyl ether), lower aliphatic alkanes (e.g. pentane, heptane) and/or, preferably, aqueous solvents, such as water. Mixtures of any of the above-mentioned solvents may be used.

There is further provided a process for the preparation of a formulation of the invention, which process comprises bringing Compound A, Compound B, Compound C, Compound D, or a pharmaceutically-acceptable salt of any of these compounds, into association with a pharmaceutically-acceptable diluent or carrier. The term "bringing into association with" includes, for example, adding one ingredient to the other, optionally with manual and/or mechanical mixing.

Formulations of the invention that are in the form of immediate release tablets may be prepared by bringing active ingredient into association with diluent/carrier using standard techniques, and using standard equipment, known to the skilled person, including wet or dry granulation, direct compression/compaction, drying, milling, mixing, tabletting and coating, as well as combinations of these processes, for example as described hereinafter.

For parenteral formulations of the invention, it is preferred that the formulations of the invention comprising active ingredient in the form of free base are prepared by adding free base (Compound A, B, C or D) to an appropriate diluent/carrier (e.g. solvent system comprising water). It is preferred that such formulations of the invention comprising active ingredient in the form of an acid addition salt are prepared by addition of acid (either directly or in association with a diluent/carrier, such as in the form of a solution (such as an aqueous solution) of appropriate acid) to base (Compound A, Compound B, Compound C or Compound D), which base may, for example be provided in association with a diluent/carrier, such as in the form of a solution (such as an aqueous solution), followed by addition of further diluent/carrier/solvent, if and as appropriate.

The skilled person will appreciate that appropriate additional excipients may be added at a suitable stage in the preparation of the formulation of the invention (i.e. before, during or after the process of bringing active ingredient into association with diluent/carrier). For example, for parenteral formulations of the invention in the form of aqueous solutions, the pH of the mixture of Compound A, B, C or D or salt thereof and diluent/carrier may be controlled by addition of an appropriate buffer and/or adjusted by way of a pH adjusting agent; for example as described hereinafter.

Preferably, the formulations of the invention comprise Compound D or a pharmaceutically acceptable salt thereof, or Compound A or a pharmaceutically acceptable salt thereof. Particularly preferred formulations of the invention include Compound D in the form of a methanesulphonate salt, tartrate salt, citrate salt, acetate salt, or in the form of the free base.

Formulations of the invention (particularly those suitable for eventual parenteral administration) in the form of a suspension, or particularly a solution, such as an aqueous solution, may be provided in "ready-to-use" form, by which we include in a form that is suitable for administration directly to a patient, with the aid of a suitable dosing means, without further preparative or formulative work being necessary.

However, such formulations (i.e. those in the form of a suspension or solution, particularly an aqueous solution) may also be provided in the form of a "concentrate" of active ingredient and diluent/carrier. Formulations in this form, hereinafter referred to as "concentrated formulations of the invention" or "concentrates", may thus be employed in the preparation of a corresponding formulation of the invention suitable for e.g. parenteral administration by addition of further diluent/carrier (and, if appropriate, further excipients), prior to administration to a patient. For example, aqueous concentrates, particularly for use in parenteral formulations, may be prepared ready for reconstitution and/or dilution (e.g. by addition of water, physiological saline, glucose solution or any other suitable solution) prior to administration.

Concentrated formulations of the invention may be prepared directly by bringing diluent or carrier (and, if appropriate, additional excipients) into association with the active ingredient as described hereinbefore. Concentrates may also be prepared by preparation of a formulation of the invention in the form of e.g. an aqueous solution, as hereinbefore described, which may include additional excipients, followed by removal of pharmaceutically-acceptable diluent or carrier (e.g. solvent, such as aqueous solvent). Solvents may be removed by way of a variety of techniques known to those skilled in the art, for example evaporation (under reduced pressure or otherwise). Thus, formulations of the invention (e.g. parenteral formulations) in ready-to-use form may also be provided by addition of diluent or carrier (and, if appropriate, further excipients) to a concentrated formulation of the invention.

The amount of diluent/carrier in an oral (e.g. immediate release tablet) formulation of the invention depends upon many factors, such as the nature and amount of the active ingredient that is employed and the nature, and amounts, of any other constituents (e.g. further excipients) that are present in the formulation, but is typically up to 40% (w/w), preferably up to 30%, more preferably up to 20%, and particularly up to 10% (w/w) of the final composition. The amount of additional excipients in such an oral formulation of the invention also depends upon factors, such as the nature and amount of the active ingredient that is employed, as well as the nature, and amounts, of any other constituents (e.g. diluents/carriers and/or other further excipients) that are present in the formulation, but, for lubricants and glidants is typically up to 5% (w/w), and for binders and disintegrants is typically up to 10% (w/w) of the final composition.

The amount of diluent/carrier in a "ready-to-use" parenteral formulation of the invention as defined above depends upon many factors, such as the nature and amount of the active ingredient that is employed and the nature, and amounts, of any other constituents (e.g. further excipients) that are present in the formulation, but is typically at least 50% (w/w) of the final composition. In concentrated formulations of the invention, the amount of diluent/carrier is typically at least 10% (w/w) of the concentrate. (It is to be noted, however, that although these lower limits for diluent/carrier content are typical, they may not always be applicable, for example in cases where the solubility of active ingredient in the relevant diluent/carrier is particularly high.)

Compositions including active ingredient may also be provided in solid form suitable for use in the preparation of a formulation of the invention (e.g. a solution, such as an aqueous solution, for example for parenteral adminstration) *ex tempore.*

Such compositions may be in the form of a solid comprising active ingredient, optionally in the presence of one or more further excipients as hereinbefore defined and, optionally, up to 10% (w/w) of diluent and/or carrier as hereinbefore defined, which compositions are hereinafter referred to as "the solid compositions of the invention".

Solid compositions of the invention may be made by removal of diluent/carrier (e.g. solvent) from a formulation of the invention, or a concentrated formulation of the invention, which may for example be in the form of a solution, such as an aqueous solution.

There is thus provided a process for the formation of a solid composition suitable for use in the preparation of a formulation of the invention (e.g. a solution, such as an aqueous solution) *ex tempore,* which process comprises removal of diluent/carrier (e.g. solvent) from a formulation of the invention, or a concentrated formulation of the invention.

Solvent may be removed by way of a variety of techniques known to those skilled in the art, for example evaporation (under reduced pressure or otherwise), freeze-drying, or any solvent removal (drying) process that removes solvent (such as water) while maintaining the integrity of the active ingredient. Freeze-drying is preferred.

Thus according to a further aspect of the invention there is provided a freeze-dried (lyophilised) solid composition of the invention.

In the preparation of solid compositions of the invention, the skilled person will appreciate that appropriate additional excipients may be added at a suitable stage prior to removal of diluent/carrier. For example, in the case of aqueous solutions, pH may be controlled and/or adjusted as hereinbefore described. Furthermore, an appropriate additional excipient may be added with a view to aiding the formation of a solid composition of the invention during the process of diluent/carrier removal (e.g. mannitol, sucrose, glucose, mannose or trehalose).

It is preferred that solid compositions of the invention comprising Compounds A, B, C or D each in the form of the free base, or Compound A, benzenesulphonic acid salt are provided in a form in which either one or more further excipients, or up to 10% (w/w) diluent/carrier, are also included in the composition.

Solid compositions of Compounds A, B, C or D, or salts thereof, thus include those in which the solvent (e.g. water) content, other than solvents of crystallisation is no more than 10%, such as less than 2% unbound solvent, such as water.

Formulations of the invention may be sterilised, for example by sterile filtration or autoclavation, and/or filled into primary packages, such as vials, cartridges and pre-filled syringes. Such processing steps may also take place prior to drying to form a solid composition of the invention.

Before administration, the dried solid composition may be reconstituted and/or diluted in, for instance, water, physiological saline, glucose solution or any other suitable solution.

The formulations of the invention are useful in the delivery of Compound A, Compound B, Compound C and Compound D, and pharmaceutically-acceptable salts of any of these compounds, to patients. As Compounds A, B, C and D, and pharmaceutically-acceptable salts thereof, are useful in both the prophylaxis and the treatment of cardiac arrhythmias, in particular atrial and ventricular arrhythmias (such as atrial fibrillation (e.g. atrial flutter)), the formulations of the invention are also expected to be useful in the treatment of such disorders.

The formulations (and indeed the concentrated formulations and solid compositions) of the invention are thus indicated in the treatment or prophylaxis of cardiac diseases, or in indications related to cardiac diseases, in which arrhythmias are believed to play a major role, including ischaemic heart disease, sudden heart attack, myocardial infarction, heart failure, cardiac surgery and thromboembolic events. Such formulations/concentrated formulations/solid compositions are also useful in such treatment where, for example, a rapid therapeutic response is required (e.g. in the treatment of acute problems), or, in the case of parenteral administration, when peroral delivery to the gastrointestinal tract is incapable of providing sufficient systemic uptake within the required time-frame.

According to a further aspect of the invention, there is provided a method of treatment of an arrhythmia which method comprises administration of a formulation of the invention to a person suffering from, or susceptible to, such a condition.

For the avoidance of doubt, by "treatment" we include the therapeutic treatment, as well as the prophylaxis, of a condition.

Formulations of the invention have the advantage that they may provide an immediate release of Compound A, Compound B, Compound C, Compound D or a pharmaceutically-acceptable salt of any of these compounds, which compound/salts are effective against cardiac arrhythmias.

Formulations of the invention may also have the advantage that they may be prepared using established pharmaceutical processing methods and employ materials that are approved for use in foods or pharmaceuticals or of like regulatory status.

Suitable amounts of active ingredient in formulations (oral or parenteral), concentrated formulations, and solid compositions, of the invention depend upon many factors, such as the nature of that ingredient (free base/salt etc), the dose that is required in an oral formulation or in a final "ready to use" parenteral formulation that is, or is to be, prepared, and the nature, and amounts, of other constituents of the formulation. However, typical daily doses of Compound A, Compound B, Compound C, Compound D, or pharmaceutically-acceptable salts thereof, are in the range 10 to 2000 mg, e.g. 30 to 1200 mg of free base (i.e., in the case of a salt, excluding any weight resulting from the presence of a counter ion), irrespective of the number of individual doses that are administered during the course of that day. (The skilled person will appreciate that in the case of immediate release parenteral formulations, such as those of the invention, administration may be continuous (e.g. by way of infusion).) Preferred daily doses are in the range 50 to 1000 mg.

### Examples

The invention is illustrated by way of the following Examples.

### Preparation A

### Preparation of Compound A and Benzenesulphonate Salt Thereof

### (i) 4-[(3-Hydroxypropyl)amino]benzonitrile

Alternative 1 A mixture of 4-fluorobenzonitrile (12.0 g, 99.1 mmol) and 3-amino-1-propanol (59.6 g, 793 mmol) was stirred at 80°C under an inert atmosphere for 3. hours before water (150 mL) was added. The mixture was allowed to cool to room temperature, and was then extracted with diethyl ether. The organic layer was separated, dried (Na₂SO₄), filtered and concentrated *in vacuo* to yield 17 g (97%) of the sub-title compound as an oil that crystallised upon standing.
Alternative 2 4-Fluorobenzonitrile (24.6 g, 0.203 mol, Aldrich 99%) was added to 3-amino-1-propanol (122.0 g, 1.625 mol, 8 equiv., Aldrich 99%) and the mixture heated to 80°C for 5 hours, under nitrogen. The solution was allowed to cool to 22°C and water (300 mL) was added. The cloudy solution was extracted twice with methylene chloride (300 mL and 200 mL) and the combined methylene chloride extracts were washed with water (300 mL; GC analysis of organic layer gave ~1.0 area% aminopropanol remaining).
Alternative 3 To 4-fluorobenzonitrile (30.29 g, 247.7 mmol, 1.0 eq), was added 3-amino-1-propanol (150 mL, 148.8 g, 1981.5 mmol, 8.0 eq). The mixture was stirred under nitrogen at room temperature (27°C) until all of the solid had dissolved. The solution was heated (oil bath) to 77°C and kept at this temperature for 7 hours, before being stirred at ambient temperature overnight (14 hours). Water (365 mL) was added, and the resultant cloudy solution was extracted with dichloromethane (365 mL, then 245 mL). The combined organic layers were washed with water (365 mL). The DCM solution of the product was dried by distillation: solvent (200 mL) was removed and replaced with fresh DCM (200 mL). More solvent (250 mL) was removed to bring the total solvent volume to 365 mL.

### (ii) 3-(4-Cyanoanilino)propyl 4-methylbenzenesulfonate

Alternative I A cooled (0°C) solution of 4-[(3-hydroxypropyl)-amino]benzonitrile (from step (i) (Alternative 1) above; 17 g, 96.5 mmol) in dry MeCN (195 mL) was treated with triethylamine (9.8 g, 96.5 mmol) and then p-toluenesulfonyl chloride (20:2 g, 106 mmol). The mixture was stirred at 0°C for 90 minutes before being concentrated *in vacuo.* Water (200 mL) was added to the residue, and the aqueous solution was extracted with DCM. The organic phase was dried (Na₂SO₄), filtered and concentrated *in vacuo.* The resulting residue was purified by crystallisation from *iso*-propanol to yield 24.6 g (77%) of the title compound.
Alternative II The solution of the crude 4-[(3-hydroxypropyl)amino]-benzonitrile (from step (i) (Alternative 2) above) was concentrated to a. volume of 300 mL by distillation and a further 200 mL methylene chloride added and re-distilled to 300 mL (solution water by Karl-Fischer 0.07%). Triethylamine (20.55 g, 0.203 mol), followed by 4-(*N,N*-dimethylamino)pyridine (248 mg, 2.0 mmol) was added and the solution was cooled to 0°C. A solution of tosyl chloride (38.70 g, 0.203 mol) in methylene chloride (150 mL) added over *ca.* 30 minutes with cooling and good agitation, allowing the temperature to rise to 5°C. The reaction was stirred for 23 hours in the range 3 to 5°C under nitrogen. (After 5 hours, triethylamine hydrochloride precipitation occurred. TLC showed very little if any further conversion of residual cyano alcohol at 20-23 hours.) Water (300 mL) was added and the layers vigorously agitated for 15 min. The organic solution was concentrated by distillation at 35 to 40°C to a volume of ca. 60 to 70 mL. *iso*-Propanol (100 mL) was added over 5 minutes. (At this stage, some granular precipitation of product occurred prior to addition of *iso*-propanol. Crystallization occurred rapidly upon addition of *iso-*propanol.) Distillation was continued using vacuum to remove the last of the methylene chloride. (A further ~30 mL was removed and the distillate was checked by GC for the absence of methylene chloride.) The crystal slurry was cooled to 0 to 5°C over *ca.* 1 hour with slow agitation and held for one hour at 0-5°C. The crystals were filtered on a medium sinter and the compacted damp filter cake carefully washed with cold (0°C) *iso*-propanol (80 mL). The filter cake was dried under vacuum and a stream of nitrogen overnight. Yield : 52.6 g, 78.4 mole% ; HPLC : 99.64 area%.
Microanalysis : found (theory) : %C :61.60 (61.67); %H :5.41 (5.49); %N : 8.44 (8.47); %S : 9.71(9.70).

### (iii) N,N-Bis(2-oxiranylmethyl)benzenesulphonamide

Water (2.5 L, 10 vol.) followed by epichlorohydrin (500 mL, 4 eq.) were added to benzenesulphonamide (250 g, 1 eq.). The reactants were heated to 40°C. Aqueous sodium hydroxide (130 g in 275 mL of water) was added such that the temperature of the reaction remained between 40°C and 43°C. This took approximately 2 hours. (The rate of sodium hydroxide addition needs to be slower at the start of the addition than at the end in order to keep within the temperature range stated.) After the addition of sodium hydroxide was complete, the reaction was stirred at 40°C for 2 hours, then at ambient temperature overnight. The excess epichlorohydrin was removed as a water azeotrope by vacuum distillation (ca. 40 mbar, internal temp 30°C), until no more epichlorohydrin distilled. Dichloromethane (1L) was added and the mixture stirred rapidly for 15 minutes. The phases were allowed to separate (this took 10 minutes although totally clear phases are obtained after standing overnight). The phases were separated and the dichloromethane solution used in the subsequent step below.
¹H NMR (400MHz, CDCl₃): δ 2.55-2.65 (2H, m), 2.79 (2H, t, *J* 4.4), 3.10-3.22 (4H, m), 3.58-3.73 (2H, m), 7.50-7.56 (2H, m), 7.58-7.63 (1H, m), 7.83-7.87 (2H, m).

### (iv) 5-Benzyl-3,7-dihydroxy-1-phenylsulphonyl-1,5-diazacyclooctane

IMS (2.5 L, 10 vol) was added to the dichloromethane solution from step (iii) above. The solution was distilled until the internal temperature reached 70°C. Approximately 1250 mL of solvent was collected. More IMS (2.5 L, 10 vol) was added followed by benzylamine (120 mL, 0.7 eq.) in one portion (no exotherm seen), and the reaction was heated at reflux for 6 hours (no change from 2 hour sampling point). More benzylamine was added (15 mL) and the solution was heated for a further 2 hours. The IMS was distilled off (ca. 3.25 L) and toluene was added (2.5 L). More solvent was distilled (ca. 2.4 L) and then further toluene added (1 L). The head temperature was now 110°C. A further 250 mL of solvent was collected at 110°C. Theoretically, this left the product in ca. 2.4 L of toluene at 110°C. This solution was used in the next step.
¹H NMR (400 MHz, CDCl₃): δ 7.83-7.80 (4H, m, ArH), 7.63-7.51 (6H, m, ArH), 7.30-7.21 (10H, ArH), 3.89-3.80 (4H, m, CH(a) +CH(b)), 3.73 (2H, s, CH₂Ph(a)), 3.70 (2H, s, CH₂Ph(b)), 3.59 (2H, dd, CHHNSO₂Ar(a)), 3.54 (2H, dd, CHHNSO₂Ar(b)), 3.40 (2H, dd, CHHNSO₂Ar(b)), 3.23 (2H, dd, CHHNSO₂Ar(a)), 3.09-2.97 (4H, m, CHHNBn(a) + CHHNBn(b)), 2.83 (2H, dd, CHHNBn(b)), 2.71 (2H, dd, CHHNBn(a))
(Data taken from purified material comprising a 1:1 mixture of *trans-* (a), and *cis*-diol (b))

### (v) 3-Benzyl-7-(phenylsulphonyl)-9-oxa-3,7-diazabicyclo[3,3,1]nonane

The toluene solution from the previous step (iv) above was cooled to 50°C. Anhydrous methanesulphonic acid (0.2 L) was added. This caused a temperature rise from 50°C to 64°C. After 10 minutes, methanesulphonic acid was added (1 L) and the reaction heated to 110°C for 5 hours. Toluene was then distilled from the reaction; 1.23 L was collected. (Note that the internal temperature should not be allowed higher than 110°C at any stage otherwise the yield will be decreased.) The reaction was then cooled to 50°C and a vacuum applied to remove the rest of the toluene. Heating to 110°C and 650 mbar allowed a further 0.53 L to be removed. (If the toluene can be removed at a lower temperature and pressure then that is beneficial.) The reaction was then left to cool to 30°C and deionised water (250 mL) was added. This caused the temperature to rise from 30°C to 45°C. More water (2.15 L) was added over a total time of 30 minutes such that the temperature was less than 54°C. The solution was cooled to 30°C and then dichloromethane (2 L) was added. With external cooling and rapid stirring, the reaction mixture was basified by adding aqueous sodium hydroxide (10 M, 2 L) at a rate that kept the internal temperature below 38°C. This took 80 minutes. The stirring was stopped and the phases separated in 3 minutes. The layers were partitioned. IMS (2 L) was added to the dichloromethane solution and distillation started. Solvent (2.44 L) was collected until the head temperature reached 70°C. Theoretically, this left the product in 1.56 L of IMS. The solution was then allowed to cool to ambient temperature overnight with slow stirring. The solid product that precipitated was filtered and washed with IMS (0.5 L) to give a fawn-coloured product that, on drying at 50°C, in vacuum, gave 50.8 g (8.9% over 3 steps). 20.0 g of this product was dissolved in acetonitrile (100 mL) at reflux to give a pale yellow solution. After cooling to ambient temperature, the crystals that formed were collected by filtration and washed with acetonitrile (100 mL). The product was dried *in vacuo* at 40°C for 1 hour to give 17.5 g (87%) of sub-title compound.
¹H NMR (400 MHz, CDCl₃): δ 7.18-7.23 (10H, m), 3.86-3.84 (2H, m), 3.67 (2H, d), 3.46 (2H, s), 2.91 (2H, d), 2.85 (2H, dd), 2.56 (2H, dd)

### (vi) 3-Benzyl-9-oxa-3,7-diazabicyclo[3.3.1]nonane x 2 HCl

Concentrated hydrobromic acid (1.2 L, 3 rel. vol.) was added to solid 3-benzyl-7-(phenylsulphonyl)-9-oxa-3,7-diazabicyclo[3.3.1]nonane (400 g, see step (v) above) and the mixture was heated to reflux under a nitrogen atmosphere. The solid dissolved in the acid at 95°C. After heating the reaction for 8 hours, HPLC analysis showed that the reaction was complete. The contents were cooled to room temperature. Toluene (1.2 L, 3 rel. vol.) was added and the mixture stirred vigorously for 15 minutes. Stirring was stopped and the phases were partitioned. The toluene phase was discarded along with a small amount of interfacial material. The acidic phase was returned to the original reaction vessel and sodium hydroxide (10 M, 1.4 L, 3.5 rel. vol.) was added in one portion. The internal temperature rose from 30°C to 80°C. The pH was checked to ensure it was >14. Toluene (1.6 L, 4 rel. vol.) was added and the temperature fell from 80°C to 60°C. After vigorous stirring for 30 minutes, the phases were partitioned. The aqueous layer was discarded along with a small amount of interfacial material. The toluene phase was returned to the original reaction vessel, and 2-propanol (4 L, 10 rel. vol.) was added. The temperature was adjusted to between 40°C and 45°C. Concentrated hydrochloric acid (200 mL) was added over 45 minutes such that the temperature remained at between 40°C and 45°C. A white precipitate formed. The mixture was stirred for 30 minutes and then cooled to 7°C. The product was collected by filtration, washed with 2-propanol (0.8 L, 2 rel vol.), dried by suction and then further dried in a vacuum oven at 40°C. Yield = 297 g (91%).
¹H NMR (CD₃OD + 4 drops D₂O): δ 2.70 (br d, 2H), 3.09 (d, 2H), 3.47 (br s, 4H), 3.60 (s, 2H), 4.12 (br s, 2H), 7.30-7.45 (m, 5H).
API MS: *m*/*z* = 219 [C₁₃H₁₈N₂O⁺H]⁺.

### (vii) 3,3-Dimethyl-1-[9-oxa-7-(phenylmethyl)-3,7-diazabicyclo[3.3.1]non-3-yl]-2-butanone

Water (500 mL, 5 vol.) followed by 1-chloropinacolone (45.8 mL, 1 eq.) were added to sodium bicarbonate (114.2 g, 4 eq.). A solution of 3-benzyl-9-oxa-3,7-diazabicyclo[3.3.1]nonane x 2 HCl (100.0 g; see step (vi) above) in water (300 mL, 3 vol.) was added slowly, so that the evolution of carbon dioxide was controlled (20 mins.). The reaction mixture was heated at 65 to 70°C for 4 hours. After cooling to ambient temperature, dichloromethane (400 mL, 4 vol.) was added and, after stirring for 15 minutes, the phases were separated. The aqueous phase was washed with dichloromethane (400 mL, 4 vol.) and the organic extracts combined. The solution was distilled and solvent collected (550 mL). Ethanol (1 L) was added and the distillation continued. Further solvent was collected (600 mL). Ethanol (1 L) was added and the distillation continued. Further solvent was collected (500 mL) (the head temperature was now 77°C). This solution (theoretically containing 1150 mL of ethanol) was used directly in the next step.
¹H NMR (400MHz, CDCl₃): δ 1.21 (9H, s), 2.01-2.59 (2H, m), 2.61-2.65 (2H, m), 2.87-2.98 (4H, m), 3.30 (2H, s), 3.52 (2H, s), 3.87 (2H, br s), 7.26 (2H, d, *J*7.6), 7.33 (1H, dd, *J*7.6, 7.6), 7.47 (2H, d, *J*7.6).

### (viii) 3,3-Dimethyl-1-(9-oxa-3,7-diazabicyclo[3.3.1]non-3-yl)-2-butanone

Palladium on charcoal (44 g, 0.4 wt. eq. of 61% wet catalyst, Johnson Matthey Type 440L) was added to the ethanol solution from the previous step (vii) above. The mixture was hydrogenated at 4 bar. The reaction was considered complete after 5 hours. The catalyst was removed by filtration and washed with ethanol (200 mL). The combined ethanol filtrates were/may be used in step (ix) below. Solution assay gave 61.8 g of title product in ethanol (theoretically 1.35 L; measured 1.65 L). A portion of the product was isolated and purified. Analysis was performed on the purified product.
¹H NMR (300MHz, CDCl₃): δ 1.17 (9H, s), 2.69 (2H, dt, *J* 11.4,2.4),2.93 (2H, d, *J* 10.8), 3.02 (2H, d, *J* 13.8), 3.26 (2H, s), 3.32 (2H, dt, *J* 14.1), 3.61 (2H, br s).

This reaction may also be performed using a lower weight ratio of catalyst to benzylated starting material. This may be achieved in several different ways, for example by using different catalysts (such as Pd/C with a metal loading different from that in the Type 440L catalyst employed above, or Rh/C) and/or by improving the mass transfer properties of the reaction mixture (the skilled person will appreciate that improved mass transfer may be obtained, for example, by performing the hydrogenation on a scale larger than that described in the above reaction). Using such techniques, the weight ratio of catalyst to starting material may be reduced below 4:10 (e.g. between 4:10 and 1:20.).

### (ix) Compound A, benzenesulphonic acid salt monohydrate

### Method 1

Potassium carbonate (56.6 g, 1.5 equiv) and 3-(4-cyanoanilino)propyl-4-methylbenzenesulphonate (see step (ii) above, 90.3 g, 1 equiv) were added to an ethanol solution of 3,3-dimethyl-1-(9-oxa-3,7-diazabicyclo[3.3.1]non-3-yl)-2-butanone (see step (viii) above; 61.8 g from assay in 1.65 L). The reaction was heated at 80°C for 4 hours. An assay showed some reactant remained (8.3 g), so more 3-(4-cyanoanilino)propyl-4-methylbenzenesulphonate (12.2 g) was added, and the resultant was heated at 80°C for 4 hours. Solvent (1.35 L) was distilled, then *iso*-propyl acetate (2.5 L) added. Solvent (2.51 L) was removed. *iso*-Propyl acetate (2.5 L) was added. Solvent (0.725 L) was removed. The internal temperature was now at 88°C. Solvent (0.825 L) was removed, leaving the product as an *iso-*propyl acetate solution (theoretically in 2.04 L). After cooling to 34°C, water (0.5 L) was added. There was a black suspension, possibly of Pd, in the mixture. The pH of the aqueous phase was 11. Sodium hydroxide (1 M, 0.31 L) was added, so that the temperature was less than 25°C, and the mixture was stirred vigourously for 5 minutes. The pH of the aqueous phase was 12. The phases were separated and the aqueous phase discarded. More water (0.5 L) was added, and the phases were separated. The aqueous phase was discarded. The remaining ester solution was filtered to remove suspended particles, and the filtrate was then made up to exactly 2 L. The solution was then split into 2 x 1 L portions.

(In order to avoid producing sub-title product comprising a high palladium content, the following treatment may be performed: Deloxan® resin (12.5 g, 25 wt%) was added to the solution of the free base (1 L), and the mixture heated at reflux with vigorous stirring for 5 hours. The solution, was then cooled to room temperature, and was stirred for 2 days. The resin was removed by filtration.)

An assay was performed to calculate the required amount of benzenesulphonic acid, to make the benzenesulphonate salt.

A solution of benzenesulphonic acid (20.04 g, 1 eq., assuming acid was pure monohydrate) in isopropyl acetate (200 mL) was added over 5 minutes (better to add slower if possible) with vigorous stirring to the solution of the free base (1 L) and a pale yellow precipitate formed. The temperature rose from 18°C to 22°C. After 10 minutes, the mixture was cooled to 10°C and the product collected by filtration. The product was washed with *iso-*propyl acetate (250 mL), sucked dry on the filter then dried under vacuum at 40°C for 2 days to give 59.0 g (61% from 3-benzyl-9-oxa-3,7-diazabicylo[3.3.1]nonane x 2HCl).

(The crude benzenesulphonate salt was alternatively prepared by the addition of a 70% (w/w) aqueous solution of benzenesulphonic acid to an ethanolic solution of the free base.)

The crude sub-title product is isolated as a monohydrate.

Ethanol (500 mL) and water (250 mL) were added to crude sub-title compound (50.0 g). The solution was heated to 75°C. Material was all dissolved at 55°C. The solution was held at 75°C for 5 minutes, then cooled to 5°C over 1 hour. Precipitation started at 18°C. The cold solution was filtered and the filtrate washed with ethanol:water (2:1; 150 mL), sucked dry on the filter, and then dried *in vacuo* at 40°C to give pure sub-title product (41.2 g, 82%).

(This recrystallisation may be carried out with greater volumes of solvent if necessary to fit the reaction vessels e.g.
EtOH : water 2:1, 45 vol. (gave 62% recovery)
EtOH : water 6:1, 35 vol. (gave 70% recovery).)

The sub-title product was isolated as the monohydrate following the rescrystallisation (as determined by single crystal X-ray diffraction).

### Method 2

### (a) 3-(4-Cyanoanilino)propyl benzenesulfonate

To the solution of 4-[(3-hydroxypropyl)amino]benzonitrile (from step (i) Alternative 3 above, assumed 43.65 g, 247.7 mmol, 1.0. eq) in dichloromethane (360 mL total solution volume) was added, sequentially, triethylamine (52 mL, 37.60 g, 371.55 mmol, 1.5 eq) and trimethylamine hydrochloride (11.89 g, 123.85 mmol, 0.5 eq) in one portion. The yellow solution was cooled to -20°C (using an acetone/dry ice bath or a cold plate), and treated with a solution of benzenesulfonyl chloride (32 mL, 43.74 g, 247.7 mmol, 1.0 eq) in dichloromethane (220 mL, 5 vols with respect to the cyanoalcohol) via a pressure equalising dropping funnel. The solution was added portionwise such that the internal temperature did not exceed -14°C. The addition took 25 minutes to complete. The mixture was then stirred for 35 minutes at between -15 and -10°C. Water (365 mL) was added and the temperature rose to 10°C. The mixture was cooled back to 0°C and stirred vigorously for 15 minutes. The organic layer (volume 570 mL) was collected and distilled at atmospheric pressure to remove DCM (450 mL, pot temperature 40-42°C, still-head temperature 38-39°C). Ethanol (250 mL) was added, and the solution was allowed to cool to below 30°C before turning on the vacuum. More solvent was removed (40 mL was collected, pressure 5.2 kPa (52 mbar), pot and still-head temperatures were 21-23°C), and the product gradually came out of solution. The distillation was stopped at this point, and more ethanol (50 mL) was added. The mixture was warmed (hot water bath at 50°C) to 40°C to dissolve all the solid, and water (90 mL) was added slowly via a dropping funnel. The solution was stirred slowly at room temperature (20°C) overnight (15 hours), by which time some product had crystallised out. The mixture was cooled to -5°C (ice/methanol bath) and stirred at this temperature for 20 minutes before collecting the pale yellow solid by filtration. The solid was washed with an ethanol/water mixture (42 mL EtOH, 8 mL H₂O), and suction dried for 30 minutes before drying to constant weight in the vacuum oven (40°C, 72 hours). The mass of crude product obtained was 47.42 g (149.9 mmole, 60%). Ethanol (160 mL, 8 vols) was added to the crude product (20.00 g, 63.22 mmol, 1.0 eq). The mixture was stirred under nitrogen and warmed to 40°C using a hot water bath. On reaching this temperature, all of the solid had dissolved to give a clear, yellow solution. Water (60 mL, 3 vols) was added dropwise over a period of 10 minutes, whilst the internal temperature was maintained in the range 38-41°C. The water bath was removed, and the solution was allowed to cool to 25°C over 40 minutes, by which time crystallisation had begun. The mixture was cooled to -5°C over 10 minutes, then held at this temperature for a further 10 minutes. The pale yellow solid was collected by filtration, suction dried for 10 minutes, then dried to constant weight in a vacuum oven (40°C, 15 hours). The mass of sub-title compound obtained was 18.51 g (58.51 mmol, 93% (from the crude product)).

### (b) Compound A, benzenesulphonic acid salt monohydrate

To an ethanol solution (total volume 770 mL, approx. 20 vols with respect to the amine) of 3,3-dimethyl-1-(9-oxa-3,7-diazabicyclo[3.3.1]non-3-yl)-2-butanone (assumed 34.97 g (verified by assay), 154.5 mmol, 1.0 eq; see step (viii) above) was added 3-(4-cyanoanilino)propyl benzenesulfonate (49.05 g, 154.52 mmol, 1.0 eq; see step (a) above) in one portion. The resultant mixture was heated at 74°C for 6 hours, then stirred at room temperature (20°C) for 65 hours (over the weekend; the skilled person will appreciate that the reaction will also succeed without this prolonged stirring at room temperature). Ethanol (370 mL) was removed, and water (200 mL) was added (this gave a 2:1 EtOH:H₂O mixture, total volume 600 mL). Upon adding the water, the pot temperature fell from 80°C to 61 °C. The solution was re-heated to 70°C, then allowed to cool naturally to ambient temperature overnight (19 hours), whilst stirring slowly. A solid was observed at this stage. The mixture was cooled to 0°C and then stirred at this temperature for 15 minutes before collecting the off-white solid by filtration. The solid was washed with a cold 2:1 mixture of ethanol:water (150 mL), suction dried for 1.25 hours, then oven-dried (40°C, 20 hours). The mass of crude product obtained was 57.91 g (103.3 mmol, 60%).

The crude product was found to be 98.47% pure (as determined by HPLC analysis), and was recrystallised (using the procedure detailed below) to give the sub-title compound in a purity of 99.75% (84% recovery).

### Recrystallisation procedure:

Ethanol (562 mL) and water (281 mL) were added to the crude product obtained above (56.2 g). The solution was heated to 75°C. All material dissolved at 55°C. The solution was held at 75°C for 5 minutes, before being cooled to 5°C over 1.5 hours. Precipitation started at 35°C. The cold solution was filtered and the collected precipitate was washed with ethanol: water (2:1, 168 mL). The solid material was sucked dry on the filter, before being dried *in vacuo* at 40°C to give product (47.1 g, 84%).

### (x) Compound A (free base)

### Method I

Crude benzenesulphonate salt (50.0 g, 1.0 equiv, from step (ix) above; Method 1) was added to aqueous sodium hydroxide (1M, 500 mL) washing in with dichloromethane (1.0 L, 20 vol). The combined mixture was stirred for 15 minutes. The layers were then separated and a small amount of interfacial material was left with the upper aqueous layer. Ethanol (500 mL, 10 vol) was added to the dichloromethane solution and then solvent was removed by distillation (1.25 L). The still head temperature was now at 78°C. The solution was allowed to cool to below reflux and ethanol (250 mL, 5 vol.) was added. Solvent was removed (250 mL). This warm solution was diluted with ethanol to 890 mL, 17.8 vol. (25 vol. assuming 100% conversion to free base). After heating to reflux the solution was cooled slowly. At 5°C a seed of title compound was added. Crystallisation began and the mixture was stirred at 5°C for 30 minutes. The product was collected by filtration and washed with ethanol (2 x 50 mL, 2 x 1 vol.). The product was then dried in a vacuum oven at 40°C for 60 hours to give an off-white powder (26.3 g; 74%).
¹H NMR (400 MHz, CDCl₃): δ 7.86-7.82 (2H, m), 7.39-7.32 (3H. m), 7.30-7.26 (2H, m), 6.47 (2H, m), 4.11-4.07 (4H, m), 3.70 (2H, s), 3.36-3.33 (4H m), 3.26 (2H, t), 3.12 (2H, d), 2.90 (2H, d), 2.28-2.21 (2H, m), 1.06 (9H, s). ¹³C NMR (CDCl₃): δ 24.07, 26.38, 41.52, 43.52, 56.17, 56.47, 63.17, 68.46, 96.61, 111.64, 121.03, 133.43.
MS (ES): *m*/*z* = 385.1 (M+H)⁺

### Method II

A mixture of 4-{[3-(9=oxa-3,7-diazabicyclo[3.3.1]non-3-yl)propyl]amino}-benzonitrile (see Preparation B(I)(vi) below; 5.73 g, 0.02 mol), K₂CO₃ (11.05 g, 0.08 mol) in MeCN (300 mL) was treated with 1-chloropinacolone (4.44 g, 0.032 mol). The mixture was stirred at 50°C overnight before DCM was added and the mixture filtered. The filter cake was then washed with a mixture of DCM and MeCN before the solvent was evaporated from the filtrate. The resulting residue was purified by chromatography on silica, eluting with a gradient of ethyl acetate : methanol : ammoniacal methanol (95:5:0 to 95:0:5), to give the title compound (5.8 g, 73.9%).

### Preparation B(I)

### Preparation of Compound B (Method I)

### (i) tert-Butyl 2-bromoethylcarbamate

Sodium bicarbonate (6.15 g, 0.073 mol) and di-*t*-butyl dicarbonate (11.18 g, 0.051 mol) were dissolved in a mixture of H₂O (50 mL) and dichloromethane (150 mL), then cooled to 0°C. 2-Bromoethylamine hydrobromide (10.0 g, 0.049 mol) was added slowly as a solid, and the reaction was stirred overnight at 25°C. The dichloromethane layer was separated, washed with H₂O (200 mL) and washed with a solution of potassium hydrogensulphate (150 mL, pH = 3.5). The organic layer was dried (Na₂SO₄) and concentrated *in vacuo.* The crude oil was chromatographed on silica gel, eluting with dichloromethane to afford 7.87 g (72%) of the sub-title compound as a clear, colorless oil.
¹H NMR (300 MHz, CDCl₃) δ 4.98 (bs, 1H), 3.45-3.57 (m, 4H), 1.47 (s, 9H)
API-MS: (M+1-C₅H₈O₂) 126 m/z

### (ii) 3-Benzyl-9-oxa-3,7-diazabicyclo[3.3.1]nonane x 2 HCl

This is an alternative preparation to that described in Preparation A(vi) above. A 3L, three-necked flask was equipped with a magnetic stirrer, a thermometer and a reflux condenser. Aqueous hydrobromic acid (48%, 0.76 L, 4.51 mol) was added to solid 3-benzyl-7-(phenylsulphonyl)-9-oxa-3,7-diazabicyclo[3.3.1].nonane (190 g, 0.53 mol, see Preparation A(v) above) and the mixture was heated to reflux under nitrogen. The solid dissolved at 90°C. After heating the mixture for 12 hours, GC analysis showed that the reaction was complete. The contents were cooled to room temperature. Toluene (0.6L) was added and the mixture was stirred for a few minutes. The phases were separated. The aqueous phase was returned to the original reaction vessel and aqueous sodium hydroxide (10M, 0.85 L, 8.5 mol) was added in one portion. The internal temperature rose to 80°C and the mixture was strongly basic. Toluene (0.8 L) was added when the internal temperature dropped to 55°C. After stirring vigorously for 30 minutes, the toluene phase was separated and returned to the original reaction vessel. 2-Propanol (1.9 L) was added and the internal temperature was adjusted to between 40°C and 50°C. Concentrated hydrochloric acid was added (until acidic) at such a rate to maintain the temperature between 40°C and 50°C. A white precipitate formed. The mixture was stirred for 30 minutes and then cooled to 7°C. The white powder was collected by filtration, washed with 2-propanol (0.4 L), dried by pulling air through the sample for ten minutes, and then further dried in a vacuum oven at 40°C. Yield: 130 g (84%).

### (iii) tert-Butyl 7-benzyl-9-oxa-3,7-diazabicyclo[3.3.1]nonane-3-carboxylate hydrochloride

A 5L, three-necked flask was equipped with an overhead stirrer, a thermometer and a nitrogen bubbler. Water (1.4 L), dichloromethane (1.4 L), sodium bicarbonate (150 g, 1.79 mol) and 3-benzyl-9-oxa-3,7-diazabicyclo[3.3.1]nonane x 2 HCl (130 g, 0447 mol, from step (ii) above) were all charged in order. The mixture was stirred rapidly for ten minutes and then di-*tert*-butyl dicarbonate (0.113 L, 0.491 mol) was added slowly. The mixture was stirred rapidly for three hours at room temperature. The organic layer was separated, dried with magnesium sulfate, filtered and concentrated to afford 160 g of an off-white solid. The off-white solid was charged into a 3L, three-necked flask equipped with an overhead stirrer, a thermometer and an addition funnel. Ethyl acetate (0.6 L) was charged and the clear solution was cooled to -10°C. A solution of HCl in dioxane (4 M) was added dropwise until the pH was less than 4. The hydrochloride salt precipitated and the mixture was stirred for an additional hour. The product was collected by filtration, washed with ethyl acetate (0.1 L), and dried overnight in a vacuum oven. The white crystalline product weighed 146 g (92% yield).

### (iv) tert-Butyl 9-oxa-3,7-diazabicyclo[3.3.1]nonane-3-carboxylate hydrochloride

Hydrochloride salt from step (iii) above (146 g, 0.411 mol) and 20% Pd(OH)₂-C (7.5 g) were charged to a Parr hydrogenator bottle. Methanol (0.5 L) was added and the bottle was shaken vigorously under an atmosphere of hydrogen at 3.5 bar. The reaction was monitored by GC analysis and was found to be complete after one hour: The catalyst was filtered and the filtrate was concentrated to afford an off-white crystalline product. The crude product was dissolved in hot acetonitrile (1.2 L), and then filtered while hot. The filtrate was diluted with ethyl acetate (1.2 L). The clear solution was allowed to stand overnight at room temperature. The first crop of crystals was collected and dried under vacuum to afford 52 g of sub-title compound as a white solid. The filtrate was concentrated to near dryness, then dissolved in hot acetonitrile (0.4 L), and diluted with ethyl acetate (0.4 L). A second crop of crystals (38 g) was obtained after cooling the solution to 10°C. Both crops were found to be comparable by GC analysis and ¹H NMR analyses. Combined yield: 90 g (83%).

### (v) tert-Butyl 7-[3-(4-cyanoanilino)propyl]-9-oxa-3,7-diazabicyclo[3.3.1]-nonane-3-carboxylate

The hydrochloride salt of *tert*-butyl 9-oxa-3,7-diazabicyclo[3.3.1]nonane-3-carboxylate (see step (iv) above; 1.1 g, 4.15 mmol) was mixed with MeCN (46 mL), water (2.5 mL) and K₂CO₃ (3.5 g, 25 mmol). The mixture was stirred for 4 h before CHCl₃ was added and the mixture was filtered through Celite®. The filtrate was concentrated *in vacuo* to give 0.933 g of the free base. This was then mixed with 3-(4-cyanoanilino)propyl 4-methylbenzenesulphonate (see Preparation A(ii) above; 2.1 g, 6.2 mmol) and K₂CO₃ (0.86 g, 6.2 mmol) in MeCN (18 mL). The resulting mixture was stirred overnight at 60°C before being concentrated *in vacuo.* The residue was treated with DCM (250 mL) and 1 M NaOH (50 mL). The layers were separated and the DCM layer washed twice with aqueous NaHCO₃, before being dried (Na₂SO₄) and concentrated *in vacuo.* The product was purified by flash chromatography, eluting with a gradient of toluene : ethyl acetate : triethylamine (2:1:0 to 1000:1000:1), to give 1.47 g (91%) of the sub-title compound.

### (vi) 4-{[3-(9-Oxa-3,7-diazabicyclo[3.3.1]non-3-yl)propyl]amino}benzonitrile

The sub-title compound was obtained in 96% yield using an analogous procedure to those described in Preparations C(v) and D(iii) below, using *tert-*butyl 7-[3-(4-cyanoanilino)propyl]-9-oxa-3,7-diazabicyclo[3.3.1]-nonane-3-carboxylate (from step (v) above).

### (vii) Compound B

To a solution of *tert-*butyl 2-bromoethylcarbamate (4.21 g, 0.019 mol; see step (i), above) in DMF (65 mL) was added 4-{[3-(9-oxa-3,7-diazabicyclo[3.3.1]non-3-yl)propyl]amino}benzonitrile (see step (vi) above, 4.48 g, 0.016 mol) and triethylamine (3.27 mL, 0.024 mol). The mixture was stirred overnight at 35°C and then concentrated *in vacuo.* The residue was dissolved in dichloromethane (80 mL) and washed with saturated sodium chloride. The aqueous layer was extracted with dichloromethane (1 x 150 mL). The combined organic extracts were dried (Na₂SO₄) and concentrated *in vacuo.* The crude red-brown oil was chromatographed (x2) on silica gel eluting with chloroform:methanol:conc. NH₄OH (9:1:0.02) to afford 3.75 g (56%) of the title compound.
¹H NMR (300 MHz, CD₃OD) δ 7.37-7.40 (d, *J*=8.8 Hz, 2H), 6.64-6.67 (d, *J*=8.8 Hz, 2H), 3.94 (bs, 2H), 3.21-3.31 (m, 4H), 3.01 (bs, 4H), 2.47-2.59 (m, 8H), 1.90 (bs, 2H), 1.39 (s, 9H)
¹³C NMR (75 MHz, CD₃OD) δ 158.5, 134.7, 121.9, 113.2, 97.7, 80.3, 69.2, 58.8, 58.1, 57.5, 57.3, 41.9, 38.3, 28.9, 26.2.
API-MS: (M+1) = 430 m/z

### Preparation B(II)

### Preparation of Compound B (Method II)

### (i) [2-(7-Benzyl-9-oxa-3,7-diazabicyclo[3.3.1]non-3-yl)ethyl]carbamic acid tert-butyl ester

### Alternative 1

### (a) 2-(tert-Butyloxycarbonylamino)ethyl tosylate

A solution of *p*-toluenesulfonyl chloride (28.40 g, 148 mmol) in dichloromethane (100 mL) was added dropwise over 30 minutes at 0°C to a mixture of *tert*-butyl *N*-(2-hydroxyethyl)carbamate (20 g, 120 mmol), triethylamine (18.80 g, 186 mmol) and trimethylammonium chloride (1.18 g, 12.4 mmol) in dichloromethane (120 mL). The mixture was stirred at 0°C for 1 hour then filtered, washing with dichloromethane (100 mL). The filtrate was washed with 10% citric acid (3 x 100 mL) and brine (100 mL). The organic layer was dried with magnesium sulfate and then filtered. The filtrate was concentrated under reduced pressure to give an oil. The oil was dissolved in ethyl acetate (40 mL) and then *iso*-hexane (160 mL) was added slowly. The resultant slurry was stirred at room temperature for 17 hours and then filtered. The collected solid was washed with *iso*-hexane (240 mL) to yield the sub-title compound as a colourless powder (25 g, 64%).
m.p. 64-66°C.
¹H-NMR (300MHz, CDCl₃,) δ 1.40 (9H, s), 2.45 (3H, s), 3.38 (2H, q), 4.07 (2H, t), 4.83 (1H, bs) 7.34 (2H, d), 7.87 (2H, d).
MS : m/z = 216 (MH⁺(316)-Boc).

### (b) [2-(7-Benzyl-9-oxa-3,7-diazabicyclo[3.3.1]non-3-yl)ethyl]carbamic acid tert-butyl ester

A solution of 3-benzyl-9-oxa-3,7-diazabicyclo[3.3.1]nonane dihydrochloride (see Preparation A(vi) above; 10 g, 34 mmol) in water (25 mL) was added slowly to a solution of sodium bicarbonate (10 g, 119 mmol) in water 10 mL). More water (5 mL) was added and the mixture was stirred at room temperature for 10 minutes. A solution of 2-(*tert-*butyloxycarbonylamino)ethyl tosylate (see step (a) above; 11.92 g, 37 mmol) in toluene (40 mL) was added. This mixture was then heated at 65-70°C for 7 hours before stirring at room temperature overnight. The reaction was reheated to 50°C and the phases were separated. The aqueous layer was extracted with toluene (40 mL) at 50°C. The combined organic layers were washed with saturated sodium bicarbonate (25 mL). The solvents were evaporated under reduced pressure to yield a mixture of oil and solid (13 g, >100%). Ethyl acetate (50 mL) and citric acid (10%, 25 mL) were added to a portion of the oily solid (5 g, 138 mmol). The aqueous layer was separated and the organic layer washed again with citric acid (10%, 20 mL). The aqueous layers were combined and treated with solid sodium bicarbonate until neutral. The aqueous phase was extracted with ethyl acetate (2 x 50 mL), dried over magnesium sulfate and filtered. The filtrate was evaporated to dryness under reduced pressure to give the sub-title compound as a colourless semi-solid, which solidified fully when stored in the refrigerator (4.68 g, 93%).
m.p. 58-60°C.
¹H-NMR (300MHz, CDCl₃) δ 1.46 (9H, s), 2.38-2.57 (4H, m), 2.6-2.68 (2H, m) 2.75-2.85 (4H, m), 3.22 (2H, q), 3.26 (2H, s), 3.83 (2H, bs), 6.17 (1H, bs) 7.2-7.4 (5H, m).
MS: *m*/*z* = 362 (MH⁺):

### Alternative 2

### (a) 3-(7-Benzyl-9-oxa-3,7-diazabicyclo[3.3.1]non-3-yl)ethyl]propionamide

Triethylamine (3.60 g, 35.7 mmol) was added slowly to a solution of 3-benzyl-9-oxa-3,7-diazabicyclo[3.3.1]nonane dihydrochloride (see Preparation A(vi) above; 5 g, 17 mmol) in ethanol (50 mL). Acrylamide (1.34 g, 18 mmol) was added to this mixture, which was then heated at reflux for 7 hours. The reaction mixture was then concentrated under reduced pressure. Water (50 mL) and sodium hydroxide (1 M, 150 mL) were added to the residue and the mixture extracted with ethyl acetate (2 x 200 mL). The combined organic extracts were dried over magnesium sulfate, filtered and concentrated under reduced pressure to give a colourless solid. This was recrystallised from ethyl acetate (50 mL) to give the sub-title compound (3.80 g, 76%).
m.p. 157-159°C.
¹H-NMR (300MHz, CDCl₃) δ 2.39 (2H, t), 2.42-2.61 (6H, m), 2.82-2.95 (4H, m), 3.39 (2H, s), 3.91 (2H, bs), 5.07 (1H, bs), 7.18-7.21 (2H, m), 7.25-7.39 (3H, m), 9.5 (1H, bs).
MS: m/z = 290 (MH⁺).

### (b) [2-(7-Benzyl-9-oxa-3,7-diazabicyclo[3.3.1]non-3-yl)ethyl]carbamic acid tert-butyl ester

*N*-Bromosuccinimide (6.0 g, 33 mmol) was added in portions over 1 minute to a solution of 3-(7-benzyl-9-oxa-3,7-diazabicyclo[3.3.1]non-3-yl)ethyl]-propionamide (see step (a) above; 5 g, 12 mmol) in potassium *tert*-butoxide in *tert*-butanol (1 M, 81 mL) and *tert*-butanol (20 mL). The mixture was then heated at 60-65°C for 30 minutes, The reaction was allowed to come to room temperature and then water (100 mL) was added. The mixture was extracted with ethyl acetate (2 x 50 mL). The combined organic extracts were washed with brine (50 mL), dried over magnesium sulfate, filtered (washing the filter cake with ethyl acetate (50 mL)) and then the filtrate concentrated under reduced pressure to give the sub-title compound as a brown oil (6.5 g, >100%).
¹H-NMR (300MHz, CDCl₃) δ 1.46 (9H, s), 2.4-2.58 (4H, m), 2.58-2.7 (2H, m) 2.75-2.91 (4H, m), 3.22 (2H, q), 3.28 (2H, s), 3.83 (2H, bs), 6.19 (1H, bs) 7.2-7.42 (5H, m).
MS: m/z = 316 (MH⁺).

### Alternative 3

### (a) 3-Benzyl-9-oxa-3,7-diazabicyclo[3.3.1]nonane

All volumes and equivalents are measured with respect to the amount of 3-benzyl-9-oxa-3,7-diazabicyclo[3.3.1]nonane dihydrochloride (see Preparation A(vi) above) used. Toluene (420 mL, 7 vols) and aqueous sodium hydroxide solution (2M, 420 mL, 7 vols, 4.0 eq) were added to 3-benzyl-9-oxa-3,7-diazabicyclo[3.3.1]nonane dihydrochloride (60.07 g, 206.03 mmole, 1.0 eq., see Preparation A(vi) above). The mixture was stirred under nitrogen, heated to 60°C and held at this temperature for 30 minutes by which time two clear layers had formed. The lower, aqueous layer was removed, and the toluene solution of sub-title compound (free base) was azeodried at atmospheric pressure (total volume of solvent removed = 430 mL; total volume of toluene added = 430 mL), then concentrated to a volume of 240 mL (4 vols). Karl Fischer analysis at this stage showed 0.06% water in the solution. The dried solution of sub-title compound (theoretically 44.98 g, 206.03 mmole, 1.0 eq) was used as such in a subsequent step.

### (b) 2-(tert-Butyloxycarbonylamino)ethyl 2,4,6-trimethylbenzenesulfonate

Triethylamine (65 mL, 465.3 mmole, 1.5 eq) was added in one portion to a solution of *tert-*butyl *N*-(2-hydroxyethyl)carbamate (50.11 g, 310.2 mmole, 1.0 eq.) in dichloromethane (250 mL, 5 vols). The solution was cooled to -10°C and trimethylamine hydrochloride (14.84 g, 155.1 mmole, 0.5 eq.) was added in one portion. The resultant mixture was cooled further to -15°C, stirred for 5 minutes, then treated with a solution of mesitylenesulfonyl chloride (74.74 g, 341.2 mmole, 1.1 eq) in dichloromethane (250 mL, 5 vols), over 28 minutes such that the internal temperature remained below -10°C. Once the addition was complete a precipitate had formed and the mixture was stirred at -10°C for a further 30 minutes. Water (400 mL, 8 vols) was added and all of the precipitate dissolved. The mixture was stirred rapidly for 5 minutes, and then the two layers were separated. A solvent swap from dichloromethane to *iso-*propanol was carried out by distillation at reduced pressure. Solvent was removed (450 mL) and replaced with *iso-*propanol (450 mL) (initial pressure was 450 mbar, b.p. 24°C; final pressure was 110 mbar, b.p. 36°C). At the end of the distillation, solvent (150 mL) was removed to bring the volume down to 350 mL (7 vols with respect to the amount of *tert*-butyl *N-*(2-hydroxyethyl)carbamate used). The solution was cooled to 25°C, then water (175 mL) was added slowly with stirring, causing the solution gradually to turn cloudy. No solid had precipitated at this stage. More water (125 mL) was added, and a solid precipitate started to form after about 75 mL had been added. The internal temperature rose from 25°C to 31°C. The mixture was stirred slowly and cooled to 7°C. The solid was collected by filtration, washed with iso-propanol:water (1:1, 150 mL) and dried *in vacuo* at 40°C for 21 hours to give the sub-title compound as a white crystalline solid (92.54 g, 87%).
m.p. 73.5°C
¹H-NMR (300MHz, CDCl₃) δ 1.42 (9H, s), 2.31 (3H, s), 2.62 (6H, s) 3.40 (2H, q), 4.01 (2H, t), 4.83 (1H, bs), 6.98 (2H, s)

### (c) [2-(7-Benzyl-9-oxa-3,7-diazabicyclo[3.3.1]non-3-yl)ethyl]carbamic acid tert-butyl ester, 2,4,6-trimethylbenzenesulfonic acid salt

A warm (28°C) solution of 2-(*tert*-butyloxycarbonylamino)ethyl 2,4,6-trimethylbenzenesulfonate (70.93 g, 206.03 mmole, 1.0 eq, see step (b) above) in toluene (240 mL, 4 vols) was added to a solution of 3-benzyl-9-oxa-3,7-diazabicyclo[3.3.1]nonane (44.98 g, 206.03 mmole, 1.0 eq.) in toluene (240 mL, 4 vols) (see step (a) above). The resultant solution was stirred rapidly under nitrogen, with heating at 68°C for 8 hours. The reaction was left to stir at ambient temperature for 84 hours. A thick, white solid precipitate had formed in a pale yellow solution. The mixture was cooled to +9°C, and sub-title compound was collected by filtration. The reaction vessel was washed with toluene (100 mL) and added to the filter. The filter cake was washed with toluene (150 mL). The white solid product was suction dried for 15 minutes, then dried to constant weight *in vacuo* at 40°C for 23 hours. The yield of sub-title compound obtained was 79.61 g, 141.7 mmole, 69%. The combined filtrate and washings (670 mL) were washed with aqueous sodium hydroxide solution (2M, 200 mL, 3.3 vols).

The mixture was heated to 60°C, and held at this temperature for 20 minutes with rapid stirring. The two layers were then separated. The toluene solution was concentrated to 200 mL by vacuum distillation (bp 50-54°C at 650-700 mbar; bp 46°C at 120 mbar at the end). As the distillation progressed, the solution became cloudy due to the formation of sub-title compound. It was assumed that 20% of the original amount of 3-benzyl-9-oxa-3,7-diazabicyclo[3.3.1]nonane remained in the filtrate, and so extra 2-(*tert*-butyloxycarbonylamino)ethyl 2,4;6-trimethylbenzenesulfonate (14.20 g, 41.21 mmole, 0.2 eq) was added in one portion (charged as a solid rather than as a solution in toluene). The cloudy solution was heated at 67°C for 8 hours with rapid stirring, and then left to stir at ambient temperature for 11 hours. The mixture was cooled to +8°C, and sub-title compound was collected by filtration. The reaction vessel was washed with more toluene (2 x 30 mL), and added to the filter. The white solid product was suction dried for 15 minutes, then dried to constant weight *in vacuo* at 40°C for 7 hours. The yield of sub-title compound was 23.25 g, 41.39 mmole, 20%. The combined yield of sub-title compound (a white solid) was 102.86 g, 183.11 mmole, 89%.
m.p. 190-190.5°C
¹H-NMR (300MHz, CDCl₃) δ 1.43 (9H, s), 2.17 (3H, s), 2.51 (6H, s), 2.73-2.80 (2H, m), 2.90-2.94 (4H, m), 3.14-3.22 (4H, m), 3.37 (2H, bm), 3.89 (2H, bs), 4.13 (2H, bs), 6.74 (2H, s), 7.12 (1H, bt), 7.42-7.46 (5H, m)

### (ii) [2-(9-Oxa-3,7-diazabicyclo[3.3.1]non-3-yl)ethyl]carbamic acid tert-butyl ester

Method 1: Sodium bicarbonate (0.058 g, 0.069 mmol) and 5% Pd/C (0.250 g, Johnson Matthey Type 440 paste) were added to a solution of [2-(7-benzyl-9-oxa-3,7-diazabicyclo[3.3.1]non-3-yl)ethyl]carbamic acid *tert*-butyl ester (see step (i), Alternative 1 above; 1 g, 2.77 mmol) in ethanol (10 mL). The mixture was then hydrogenated at 500 kPa (5 bar) for 18 hours. The reaction mixture was filtered through Celite® and then washed with ethanol (20 mL). The solution was concentrated under reduced pressure to give an oil. This was dissolved in dichloromethane (20 mL) and washed with sodium hydroxide (1 M, 10 mL). The organic phase was separated, dried over magnesium sulfate and then filtered. The filtrate was concentrated under reduced pressure to give the sub-title compound as a yellow solid (0.67 g, 87%).
m.p. 91-93°C.
¹H-NMR (300MHz, CDCl₃) δ 1.46 (9H, s), 2.25 (2H, t), 2.58-2.65 (2H, m) 2.95-3.06 (4H, m), 3.2-3.38 (4H, m), 3.64 (2H, bs), 4.65 (1H, bs).
MS: m/z = 272 (NM⁺).

Method 2: [2-(7-Benzyl-9-oxa-3,7-diazabicyclo[3.3.1]non-3-yl)ethyl]-carbamic acid *tert*-butyl ester 2,4,6-trimethylbenzenesulfonic acid salt (320 g, 1.0 mol eq, 1.0 rel vol/wt, see step (i), Alternative 3 above), toluene (640 mL, 2.0 vol) and aqueous sodium hydroxide (1M, 1.6 L, 5.0 vol) were stirred together for 15 minutes and the layers were then separated. The organic layer, containing [2-(7-benzyl-9-oxa-3,7-diazabicyclo[3.3.1]non-3-yl)ethyl]carbamic acid *tert*-butyl ester, was diluted with ethanol (690 mL, 2.16 vol) and water (130 mL, 0.4 vol). Citric acid (32.83g, 0.3 mol eq) and 5% Pd/C (20.8 g, 0.065 wt eq of 61% water wet catalyst, Johnson Matthey type 440L) were added. The combined mixture was then hydrogenated under 4 bar of hydrogen pressure for 24 hours. The reaction was monitored by TLC, using a silica plate with mobile phase X:DCM (1:1 v/v; X is chloroform:methanol:concentrated ammonia 80:18:2 v/v). Visualisation was by UV light (254 nm) and by staining with aqueous potassium permanganate. This showed the complete disappearance of starting material and the appearance of the sub-title compound. The reaction mixture was filtered through kieselguhr and was washed with ethanol (590 mL, 1.84 vol). The resulting solution of sub-title compound (assumed 154.85 g, 100%) was used directly in a subsequent reaction.

Method 3: [2-(7-Benzyl-9-oxa-3,7-diazabicyclo[3.3.1]non-3-yl)ethyl]-carbamic acid *tert*-butyl ester 2,4,6-trimethylbenzenesulfonic acid salt (50 g, 1.0 mol eq., 1.0 rel vol/wt, see step (i), Alternative 3 above), toluene (100 mL, 2.0 vol) and aqueous sodium hydroxide (1M, 100 L, 2.0 vol) were stirred together for 20 minutes, then at 30°C for 10 minutes, and the layers were then separated. The organic layer, containing [2-(7-benzyl-9-oxa-3,7-diazabicyclo[3.3.1]non-3-yl)ethyl]carbamic acid *tert*-butyl ester, was diluted with ethanol (100 mL, 2.0 vol.). To this was added a solution of citric acid (5.14 g, 0.3 mol eq) in water (5 mL, 0.1 vol), followed by 5% Pd/C (1.50 g, 0.03 wt eq of 61% water wet catalyst, Johnson Matthey type 440L). The combined mixture was then hydrogenated under 4 bar of hydrogen pressure for 24 hours. The reaction was monitored by TLC, using a silica plate with mobile phase X:DCM 1:1 v/v, (X is chloroform:methanol:concentrated ammonia 80:18:2 v/v). Visualisation was by UV light (254 nm) and by staining with aqueous potassium permanganate. This showed the complete disappearance of starting material and the appearance of the sub-title compound. The reaction mixture was basified with aqueous sodium hydroxide (10M, 8 mL, 0.9 mol eq), then filtered through kieselguhr. The filter-cake was washed with ethanol (100 mL, 2.0 vol). The resulting solution of sub-title compound (assumed 24.15 g, 100%) was used directly in a subsequent reaction.

### (iii) Compound B

### Method I

3-(4-Cyanoanilino)propyl-4-methylbenzenesulfonate (see Preparation A(ii) above; 0.30 g, 0.92 mmol) and potassium carbonate (0.2 g, 1.38 mmol) were added to a solution of [2-(9-oxa-3,7-diazabicyclo[3.3.1]non-3-yl)ethyl]carbamic acid *tert*-butyl ester (see step (ii), Method 1 above; 0.250 g, 0.92 mmol) in ethanol (5 mL). The reaction mixture was heated to 70°C for 10 hours before concentrating the mixture under reduced pressure. The residue was partitioned between ethyl acetate (20 mL) and sodium hydroxide (1 M, 10 mL). The aqueous phase was re-extracted with ethyl acetate (20 mL). The combined organic phases were concentrated under reduced pressure to give a yellow solid (0.290 g). The solid was dissolved in ethyl acetate (10 mL) and this solution washed with a solution of citric acid (0.250 g) in water (10 mL). The aqueous phase was separated, basified with sodium hydroxide (1 M, 10 mL) and extracted with ethyl acetate (2 x 10 mL). All organic phases were combined, dried over magnesium sulfate and then filtered (washing filtered solids with ethyl acetate (10 mL)). The filtrate was concentrated under reduced pressure to give a yellow solid (0.160 g). This was slurried in ethyl acetate (0.2 mL) and then filtered to give title compound (0.050 g, 12%).
m.p 113-115°C.
¹H-NMR (400MHz, DMSO-*D*_{*6*}) δ 1.32 (9H, s), 1.7 (2H, qt), 2.20 (2H, t), 2.22-2.3 (4H, m), 2.38-3.1 (2H, m) 2.8-2.85 (4H, m), 3.05 (2H, q), 3.19 (2H, q), 3.79 (2H, bs), 6.47 (1H, t), 6.66 (2H, d), 6.69 (1H, t), 7.41 (2H, d).
MS: m/z = 430 (MH⁺).

### Method II

To the solution of [2-(9-oxa-3,7-diazabicyclo[3.3.1]non-3-yl)ethyl]carbamic acid *tert*-butyl ester generated in step (ii) (Method 3) above (assumed 24.15 g, 1.0 mol eq., 1.0 wt./vol.) in a mixture of toluene (approx. 100 mL), ethanol (approx. 200 mL) and water (approx. 14 mL), was added anhydrous potassium carbonate (18.58 g, 1.5 mol eq.). Solid 3-(4-cyanoanilino)propyl benzenesulfonate (28.17 g, 1.0 mol eq., see Preparation A(ix), Method 2, step (a) above) was added and the combined mixture was heated to 70°C for six hours. The reaction was monitored by TLC using a silica plate with mobile phase X:DCM 1:1 v/v (in which X is chloroform:methanol:concentrated ammonia 8.0:18:2 v/v). Visualisation was by UV light (254 nm) and by staining with aqueous potassium permanganate. This showed the complete disappearance of starting material and the appearance of the title compound. The reaction mixture was cooled, and the solvent was concentrated *in vacuo.* The residue was partitioned between toluene (200 mL) and water (200 mL). The layers were separated, and the organic phase was concentrated *in vacuo* to afford a yellow solid (38.6 g). This crude material was dissolved in *iso*-propanol (190 mL, 5.0 rel. vol.) at 60°C, and the hot solution was filtered. The filtrate was stirred, and left to cool to room temperature. A white solid crystallised. The mixture was cooled from room temperature to approximately 8°C. The product was collected by filtration and was washed with *iso*-propanol (50 mL, 2.0 vol.). The damp product was dried *in vacuo* at 40°C to constant weight to give the title compound as a white crystalline solid (30.96 g, 81%).
m.p. 113.5°C
¹H-NMR (400MHz, CD₃OD) δ 1.40 (9H, s), 1.81-1.90 (2H, m), 2.35-2.54 (8H, m), 2.93 (4H, t) 3.18-3.27 (4H, m), 3.87 (2H, bs), 6.66 (2H, d), 7.39 (2H, d)
MS: m/z = (MH⁺, 430)

### Preparation C

### Preparation of Compound C

### (i) 4-(4-Cyanophenyl)but-3-yn-1-ol

Potassium carbonate (376.7 g, 2.5 mol eq.) was dissolved in a mixture of 1,2-dimethoxyethane (DME, 1.2 L, 6 vol) and water (1.2 L, 6 vol). Palladium on charcoal (20 g, 0.01 mol eq., 10% Johnson Matthey type 87L, 60% water), triphenylphosphine (11.5 g, 0.04 mol eq.) and copper(I) iodide (4.2 g, 0.02 mol eq.) were added. 4-Bromobenzonitrile (200 g, 1 mol eq.) was then added, washing in with a mixture of DME (200 mL, 1 vol) and water (200 mL, 1 vol). This mixture was stirred rapidly under nitrogen for a minimum of thirty minutes. A solution of but-3-yn-1-ol (92.1 mL, 1.1 mol eq) in DME (200 mL, 1 vol) and water (200 mL, 1 vol) was added dropwise over five minutes. The combined mixture was then heated to 80°C for three hours. The reaction was monitored by HPLC for the disappearance of arylbromide and the formation of sub-title compound. Once all of the starting material had been consumed, the reaction was cooled to 25°C and filtered through kieselguhr. The filter cake was washed separately with toluene (1.6 L, 8 vol). The DME:water mixture was partially concentrated *in vacuo* to remove the majority of the DME. This was then partitioned with the toluene wash. The toluene layer was concentrated *in vacuo* to give sub-title alkyne as a yellow solid, which was dried in a vacuum oven overnight at 40°C. Yield 182.88 g, 97%.
¹H NMR (300 MHz, CDCl₃) δ 7.599-7.575 (d, *J*=7.2 Hz, 2H, CH), 7.501-7.476 (d, *J=*7.5 Hz, 2H, CH), 3.880-3.813 (q, 2H, CH₂), 2.751-2.705 (t, 2H, CH₂), 1.791-1.746 (t, 1H, OH)
mp 79.6-80.5°C

### (ii) 4-(4-Hydroxybutyl)benzonitrile

4-(4-Cyanophenyl)but-3-yn-1-ol (40 g, 1 wt eq, see step (i) above) in ethanol (200 mL, 5 vol) and palladium on charcoal (20 g, 0.5 wt eq, 10% Johnson Matthey type 487, 60% water) were stirred rapidly under five bar hydrogen pressure for five hours. The reaction was monitored by HPLC for the disappearance of the starting material, and the formation of sub-title compound. The reaction was filtered through kieselguhr and washed with ethanol (80 mL, 2 vol). The ethanol solution was concentrated *in vacuo* to give sub-title alcohol as a yellow-brown oil. Yield 36.2g, 88.5%.
¹H NMR (300 MHz, CDCl₃) δ 7.550-7.578 (d, *J*=8.4 Hz, 2H), 7.271-7.298 (d, *J*=8.1 Hz, 2H), 3.646-3.688 (t, 2H), 2.683-2.733 (t, 2H), 1.553-1.752 (m, 4H)
¹³C NMR (300 MHz, CDCl₃) δ 148.04 (C), 132.16 (C), 119.1 (C), 109.64 (C), 62.46 (C), 35.77 (C), 32.08 (C), 27.12 (C).

### (iii) 4-(4-Cyanophenyl)butyl toluenesulphonate

The sub-title compound was prepared by addition of toluenesulphonyl chloride to 4-(4-hydroxybutyl)benzonitrile (see step (ii) above).

### (iv) tert-Butyl 7-[4-(4-cyanophenyl)butyl]-9-oxa-3,7-diazabicyclo[3.3.1]-nonane-3-carboxylate

A 2L three-necked flask was equipped with a magnetic stirrer, a thermometer and a reflux condenser. The flask was charged with a solution of 4-(4-cyanophenyl)butyl toluenesulphonate (72 g, 0.218 mol, see step (iii) above) in dimethylformamide (0.55 L). *tert*-Butyl 9-oxa-3,7-diazabicyclo[3.3.1]nonane-3-carboxylate hydrochloride (48.2, 0.182 mol, see Preparation B(I)(iv) above) was added, followed by potassium carbonate (62.9 g, 0.455 mol). The heterogeneous mixture was stirred for 22 hours at 85°C. TLC analysis indicated complete consumption of starting material.

The reaction mixture was cooled to room temperature and diluted with water (0.5 L). The mixture was extracted with ethyl acetate (3 x 0.4 L) and the organic fractions were combined. After washing with water (2 x 200 mL) and brine (200 mL), the organic layer was dried with magnesium sulfate, filtered and concentrated under vacuum. The crude brown oil was purified by chromatography on silica gel, eluting with 3:2 hexanes/ethyl acetate affording 34 g (48% yield) of sub-title compound as an off-white solid.

### (v) 4-[4-(9-Oxa-3,7-diazabicyclo[3.3.1]non-3-yl)butyl]benzonitrile

A 2L three-necked flask was equipped with a magnetic stirrer, a thermometer and an addition funnel. The flask was charged with *tert*-butyl 7-[4-(4-cyanophenyl)butyl]-9-oxa-3,7-diazabicyclo[3.3.1]-nonane-3-carboxylate (34 g, 88 mmol, from step (iv) above) and dichloromethane (440 mL). Trifluoroacetic acid (132 mL) was added slowly at room temperature. The solution was stirred for three hours at which point TLC analysis showed complete consumption of starting material. The contents were transferred to a single-necked flask and concentrated under vacuum. The residue was dissolved in dichloromethane (500 mL) and washed with saturated sodium bicarbonate solution. The aqueous layer was separated and extracted with dichloromethane (2 x 200 mL). The combined organic layers were washed with brine (200 mL), dried over magnesium sulfate and concentrated under vacuum to afford 25.8 g (100% yield) of sub-title compound as an off-white solid. The crude material was used in the next step without further purification.

### (vi) Compound C

A 3L three-necked flask was equipped with a magnetic stirrer, a thermometer and a reflux condenser. The flask was charged with impurified 4-[4-(9-oxa-3,7-diazabicyclo[3.3.1]non-3-yl)butyl]benzonitrile (25.8 g, 88 mmol, from step (v) above), dichloromethane (0.88 L) and *tert-*butyl 2-bromoethylcarbamate (see Preparation B(I)(i) above, 27.7 g, 123 mmol). Triethylamine (0.0197 L, 0.141 mol) was then added. The clear solution was refluxed for 12 hours under a nitrogen atmosphere and then cooled to room temperature. The progress of the reaction was monitored by TLC analysis and it was found to be complete at this point. The reaction mixture was transferred to a separating funnel and washed sequentially with water (200 mL), 15% aqueous sodium hydroxide (200 mL), water (200 mL), and brine (200 mL). The organic layer was dried over magnesium sulfate and concentrated under vacuum. The resulting yellow viscous oil was chromatographed on silica gel, eluting first with 9:1 dichloromethane/methanol, then with 9:1:0.02 dichloromethane/methanol/28% aqueous ammonium hydroxide to afford the title compound (25.1 g, 66% yield) as an off-white solid. The earlier fractions (5.1 g) from chromatography were found to contain a small amount of a less polar impurity (by TLC analysis) eluting with 9:1:0.05 dichloromethane/methanol/28% aqueous ammonium hydroxide) while the later factions (20 g) were one spot by TLC analysis. The earlier fractions (5.1 g) were combined with another lot of title compound (7.1 g, containing a slight impurity) and chromatographed on silica gel, eluting first with 19:1 dichloromethane/methanol, and then with 9:1 dichloromethane/methanol to afford a pale yellow powder (5.5 g). The powder was dissolved in dichloromethane (200 mL). The resulting solution was washed sequentially with 25% aqueous sodium hydroxide (50 mL), water (50 mL), and brine (40 mL). The material was then dried over magnesium sulfate and concentrated under vacuum to afford title compound as an off-white powder (5 g). The 20 g fraction was dissolved in dichloromethane (500 mL). The organic layer was washed sequentially with 25% aqueous sodium hydroxide (100 mL), water (100 mL), and brine (100 mL). The material was then dried over magnesium sulfate and concentrated under vacuum to afford title compound as an off-white powder (19 g). The lots were blended together.

### Preparation D

### Preparation of Compound D

### (i) 4-[(2S)-Oxiranylmethoxy]benzonitrile

Potassium carbonate (414 g) and (*R*)-(-)-epichlorohydrin (800 mL) were added to a stirred solution of *p*-cyanophenol (238 g) in 2.0 L MeCN and the reaction mixture was refluxed under an inert atmosphere for 2 h. The hot solution was filtered and the filtrate concentrated, giving a clear oil which was crystallised from di-*iso*-propyl ether giving the product in 90% yield.

### (ii) tert-Butyl 7-[(2S)-3-(4-cyanophenoxy)-2-hydroxypropyl]-9-oxa-3,7-diazabicyclo[3.3.1]nonane-3-carboxylate

A 3L, three-necked flask equipped with a magnetic stirrer and a thermometer was charged with *tert*-butyl 9-oxa-3,7-diazabicyclo[3.3.1]nonane-3-carboxylate as its free base (53.7 g, 0.235 mol, obtained from the hydrochloride salt, see Preparation B(I)(iv) above), 4-[(2*S*)-oxiranylmethoxy]benzonitrile (41.2 g, 0.235 mol, see step (i) above), and a 10:1 (v/v) solution of 2-propanol/water (0.94 L). The mixture was stirred at 60°C for 20 hours, during which time the starting materials were gradually consumed (assay by TLC analysis). The mixture was cooled and concentrated under vacuum to afford 100 g (>100% yield) of sub-title compound as white solid. The unpurified material was used in the next step.

### (iii) 4-{[(2S)-2-Hydroxy-3-(9-oxa-3,7-diazabicyclo[3.3.1]non-3-yl)propyl]-oxy}benzonitrile

A 3L, three-necked flask equipped with a magnetic stirrer, a thermometer and an addition funnel was charged with unpurified *tert*-butyl 7-[(2*S*)-3-(4-cyanophenoxy)-2-hydroxypropyl]-9-oxa-3,7-diazabicyclo[3.3.1]nonane-3-carboxylate (100 g, from step (ii) above) and dichloromethane (1.15 L). Trifluoroacetic acid (0.352 L) was added slowly at room temperature and the resulting solution was stirred for three hours, at which point TLC analysis showed complete reaction. The contents were transferred to a single-necked flask and concentrated under vacuum. The residue was dissolved in dichloromethane (1.2 L) and washed with saturated sodium bicarbonate. The aqueous layer was separated and extracted with dichloromethane (2 x 0.2 L). The combined organic layers were washed with brine (0.25 L), dried over magnesium sulfate and concentrated under vacuum to afford 73 g (> 100% yield) of sub-title compound as an off-white solid. The unpurified material was used in the next step.

### (iv) Compound D

Method I: A 2L, three-necked flask was equipped with a magnetic stirrer, a thermometer and a reflux condenser. The flask was charged with unpurified 4- {[(2*S*)-2-hydroxy-3-(9-oxa-3,7-diazabicyclo[3.3.1]non-3-yl)propyl]-oxy}benzonitrile (73 g, from step (iii) above), dichloromethane (0.7 L) and *tert*-butyl 2-bromoethylcarbamate (see Preparation B(I)(i) above, 74 g, 0.330 mol). Triethylamine (52 mL, 0.359 mol) was then added. The clear solution was refluxed for 16 hours and then cooled to room temperature. The reaction mixture was transferred to a separating funnel and washed sequentially with water (100 mL) and brine (100 mL). The organic layer was dried over magnesium sulfate, filtered and concentrated under vacuum. The resulting yellow viscous oil was purified by chromatography on silica gel, eluting first with 9:1 dichloromethane/methanol, then with 9:1:0.02 dichloromethane/methanol/28% aqueous ammonium hydroxide to afford an off-white foamy solid (40 g). The solid was dissolved in dichloromethane (200 mL) and washed sequentially with 20% aqueous sodium hydroxide (100 mL) and water (100 mL). The organic layer was dried over magnesium sulfate and concentrated under vacuum to afford title compound as an off-white solid (35.4 g, 67% yield in three steps).

Method II: *iso*-Propanol (5 mL) and water (0.5 mL) were added to [2-(9-oxa-3,7-diazabicyclo[3.3.1]non-3-yl)elhyl]carbamic acid *tert*-butyl ester (see Preparation B(II)(ii), Method I above; 0.43 g, 1.6 mmol) and 4-[(2*S*)-oxiranylmethoxy]benzonitrile (0.280 g, 1.6 mmol, see step (i) above) was added. The mixture was heated at 66°C for 19 hours (reaction was complete in 2 hours). The solvent was evaporated to dryness under reduced pressure to give the title compound as an off-white solid (0.71 g, 100%).
¹H-NMR (300MHz, CDCl₃) δ 1.41 (9H, s), 2.3-2.75 (6H, m), 2.75-3.0 (5H, m), 3.1-3.38 (3H, m), 3.88 (2H, s), 3.95-4.19 (3H, m), 5.85 (1H, bs), 6.99 (2H, d), 7.6 (2H, d).
¹H-NMR (300MHz, DMSO*-D*_{*6*}) *δ* 1.35 (9H, s), 2.12-2.59 (7H, m), 2.63-2.78 (1H, m), 2.78-2.9 (4H, m), 3.2 (2H, q), 3.78 (2H, m), 4-4.1 (2H, m), 4.12-4.19 (1H, m), 5.3 (1H, bs), 6.61 (1H, t), 7.15 (2H, d), 7.76 (2H, d).
MS: m/z = 447 (MH⁺).

Method III: The solution of [2-(9-oxa-3,7-diazabicyclo[3.3.1]non-3-yl)ethyl]carbamic acid *tert*-butyl ester generated in Preparation B(II)(ii), Method 2 above (assumed 154.85 g, 1.0 mol eq, 1.0 wt/vol) in a mixture of toluene (approx 640 mL), ethanol (approx 1280 mL) and water (approx 130 mL), was basified with aqueous sodium hydroxide (10M, 51 mL, 0.9 mol eq.). Solid 4-[(2S)-oxiranylmethoxy]benzonitrile (99.80g, 1.0 mol eq.; see step (i) above) was added and the combined mixture was heated to 70°C for four hours. The reaction was monitored by TLC using a silica plate with mobile phase X:DCM 1:1 v/v (in which X is chloroform:methanol:concentrated ammonia 80:18:2 v/v). Visualisation was by UV light (254 nm) and by staining with aqueous potassium permanganate. This showed the complete disappearance of starting material and the appearance of the title compound. The reaction mixture was cooled, filtered through kieselguhr and washed through with ethanol (620 mL, 4.0 vol). This gave a solution of title compound (assumed 254.38 g, 100% th, 2.4 L, 1.0 wt/vol for reaction work up). This solution was charged into a flask that was set up for reduced pressure distillation. A graduation line was marked onto the side of this flask. Solvent (1250 mL) was removed at between 50°C and 35°C, 320 mbar and 100 mbar. Then 4-methylpentan-2-ol (1500 mL) was added in order to reach the graduated line. Solvent (1250 mL) was removed at between 35°C and 80°C, 220 mbar and 40 mbar. More 4-methylpentan-2-ol (1500 mL) was added in order to reach the graduated line. Solvent (1250 mL) was removed at between 62°C and 76°C, 100 mbar and 90 mbar. The combined mixture was cooled to less than 25°C and aqueous sodium hydroxide (2M, 1.27 L, 5.0 vol) was added. The layers were separated and the organic layer was filtered through kieselguhr to give a clear solution (1.2 L). This solution was charged into a clean flask, which was set up for reduced pressure distillation. Solvent (450 mL) was removed at between 52°C and 55°C, 90 mbar and 35 mbar. Theoretically, the product was now left in 2 volumes of 4-methylpentan-2-ol. Di-n-butyl ether (1.27 L, 5 vol) was added and the solution was allowed to cool slowly to room temperature, which caused a precipitate to form. The mixture was cooled from room temperature to approximately 10°C. The product was collected by filtration and was washed with a pre-mixed solution of di-n-butyl ether (320 mL, 1.25 vol) and 4-methylpentan-2-ol (130 mL, 0.50 vol). The damp product was dried *in vacuo* at 55°C to constant weight to give the title compound as a white solid (193.6 g, 76%).
m.p. 99-101 °C
¹H-NMR (300MHz, CDCl₃) δ 1.41 (9H, s), 2.3-2.75 (6H, m), 2.75-3.0 (5H, m), 3.1-3.38(3H, m), 3.88 (2H, s), 3.95-4.19 (3H, m), 5.85 (1H, bs), 6.99 (2H, d), 7.6 (2H, d).

### Crystallisation of Compound D

A mixture of Compound D (prepared analogously to the procedures described hereinbefore (see especially Preparation D(iv), Method III above); 14.29 g), *iso*-propanol (28 mL) and di-*iso*-propyl ether (140 mL) was heated to 80°C. The solution was filtered hot to clarify it and then reheated to 80°C. The solution was then allowed to cool to room temperature whereupon a precipitate started to form. After stirring for two hours the precipitate was collected by filtration, washed with *iso*-propanol:*iso*-propyl ether (1:6, 70 mL) and then sucked dry on the filter. The damp product was dried *in vacuo* at 70°C overnight to give crystalline Compound D as a white solid (10.1 g, 70%).
¹H-NMR (300MHz, CDCl₃) δ 1.41 (9H, s), 2.3-2.75 (6H, m), 2.75-3.0 (5H, m), 3.1-3.38(3H, m), 3.88 (2H, s), 3.95-4.19 (3H, m), 5.85 (1H, bs), 6.99 (2H, d), 7.6 (2H, d)

### Preparation of Methanesulphonic Acid Salt of Compound D

The title salt was prepared by:
(a) dissolving Compound D (prepared using analogous techniques to those described above) in methanol and adding methanesulphonic acid; and
(b) dissolving Compound D in ethyl acetate and adding methanesulphonic acid as a solution in ethyl acetate, followed by seeding,
and then, in both cases, standard work up and isolation.

### Examples 1 to 10

### Aqueous Formulations

### Example 1

| | |
|---|---|
| Compound A | 60 µmol |
| Tartaric acid | 60 µmol |
| Sodium hydroxide to pH 4 | *q.s.* |
| Water | to 1.0 mL |

A formulation was prepared by dissolving Compound A in an equivalent molar amount of tartaric acid. Water was added to about 90% of the final volume. The pH was checked and adjusted to 4 by addition of aqueous sodium hydroxide. Water was added to the final volume.

This composition was given orally to rats in a 14 day toxicity study. The dose 420 µmol/kg gave plasma concentrations in the range 5.4-8.4 µM (=µmol/L) after 1 hour.

### Example 2

| | |
|---|---|
| Compound A | 10.4 µmol |
| Tartaric acid | 15.4 µmol |
| Sodium chloride | 9 mg |
| Sodium hydroxide to pH 4 | *q.s.* |
| Water | to 1.0 mL |

A formulation was prepared by dissolving Compound A in an equivalent molar amount of tartaric acid. Tartaric acid buffer was prepared by adding the remainder of the tartaric acid along with the sodium chloride to the water. The pH of the buffer was checked and adjusted to 4 by addition of aqueous sodium hydroxide. (This procedure for making buffers was also employed in certain Examples below; specific detail is not reproduced.) Buffer (5 mM) was added to about 90% of the final volume. The pH was checked and adjusted to 4 by addition of aqueous sodium hydroxide. Further tartaric acid buffer (5 mM, pH 4) was added to the final volume.

This composition was administered subcutanously to dogs for 5 days in a VTI (vaso tissue irritation) study.

### Example 3

| | |
|---|---|
| Compound A | 5 µmol |
| Acetic acid | 10 µmol |
| Sodium chloride | 9 mg |
| Sodium hydroxide to pH 5 | *q.s.* |
| Water | to 1.0 mL |

A formulation was prepared by dissolving Compound A in an equivalent molar amount of acetic acid. Acetate buffer (5 mM, pH 5, containing sodium chloride) was added to about 90% of the final volume. The pH was checked and adjusted to 5 by addition of aqueous sodium hydroxide. Acetate buffer (5mM, pH 5) was added to the final volume.

### Example 4

| | |
|---|---|
| Compound A, benzenesulphonic acid salt | 45 µmol |
| Hydroxypropylmethylcellulose (HPMC) | 5 mg |
| Water | to 1.0 mL |

A suspension of active compound was prepared by dispersing it in a HPMC 5 mg/mL solution in water, followed by mixing.

This composition was administered orally to rats in a 14 day toxicity study. A dose of 450 µmol/kg gave plasma concentrations in the range 8-12 µM (=µmol/L) after 1 hour.

### Example 5

| | |
|---|---|
| Compound A, benzenesulphonic acid-salt. | 70 µmol |
| Hydroxypropyl-β-cyclodextrin/water (40/60 w/w %) | to 1.0 mL |

Salt was dissolved in the 40/60 (w/w%) hydroxypropyl-β-cyclodextrin/water vehicle.

The solubility of the salt was almost 40 times higher in this vehicle compared to water alone.

### Example 6

| | |
|---|---|
| Compound A, benzenesulphonic acid salt | 8 µmol |
| Polyethyleneglycol 400/Ethanol/Solutol^{TM}/Water | |
| (40/10/5/45 w/w%) | to 1.0 mL |

Salt was dissolved in PEG 400, ethanol and the surfactant Solutol^{TM} (BASF; comprises polyethylene glycol esters of 12-OH-stearic acid). Water was added to the final volume. The solubility of the benzenesulphonate salt was approximately 4 times higher in this vehicle than it is in water alone.

### Example 7

| | |
|---|---|
| Compound D | 20 µmol |
| Tartaric acid | 25 µmol |
| Sodium hydroxide to pH 4 | *q.s.* |
| Water | to 1.0 mL |

A formulation was prepared by dissolving Compound D in an equivalent molar amount of tartaric acid. Tartaric acid buffer 5 mM, pH 4 was added to about 90% of the final volume. The pH was checked and adjusted to 4 by addition of aqueous sodium hydroxide. Tartaric acid buffer (5mM, pH 4) was added to the final volume.

This composition was administered orally to dogs in a 5 day dose-finding study. The dose of 40 µmol/kg gave plasma concentrations in the range 4.8-8.7 µM (=µmol/L) after 30 minutes.

### Example 8

| | |
|---|---|
| Compound D | 10.4 µmol |
| Citric acid | 15.4 µmol |
| Sodium chloride | 9.0 mg |
| Sodium hydroxide to pH 6 | *q.s.* |
| Water | to 1.0 mL |

A formulation was prepared by dissolving Compound D in an equivalent molar amount of citric acid. Citric acid buffer (5 mM, pH 6) was added to about 90% of the final volume. The pH was checked and adjusted to 6 by addition of aqueous sodium hydroxide. Citric acid buffer (5mM, pH 6) was added to the final volume.

This composition is given subcutaneously to dogs for 5 days in a VTI study.

### Example 9

| | |
|---|---|
| Compound D | 10 µmol |
| Citric acid | 15 µmol |
| Sodium chloride | 9.0 mg |
| Sodium hydroxide to pH 5 | *q.s.* |
| Water | to 1.0 mL |

A formulation was prepared by dissolving Compound D in an equivalent molar amount of citric acid. Citric acid buffer (5 mM, pH 5) was added to about 90% of the final volume. The pH was checked and adjusted to 5 by addition of aqueous sodium hydroxide. Citric acid buffer (5mM, pH 5) was added to the final volume.

### Example 10

| | |
|---|---|
| Compound D, methanesulphonic acid salt | 10 mg |
| Sodium chloride | 9 mg |
| Water | to 1 mL |

The formulation is prepared by first dissolving the sodium chloride in the water, and then dissolving Compound D, methanesulphonic acid salt in the resultant saline solution.

### Example 11

### Freeze-Dried Compositions

The following formulations are made in accordance with techniques described in one or more of Examples 1 to 10 above:
(a)

| | |
|---|---|
| Compound A | 10 mg |
| Tartaric acid | 3.9 mg |
| Mannitol | 10 mg |
| Sodium hydroxide to pH 4 | *q.s.* |
| Water | to 1 mL |

(b)

| | |
|---|---|
| Compound A | 10 mg |
| Hydrochloric acid | 0.95 mg |
| Mannitol | 10 mg |
| Sodium hydroxide to pH 4 | *q.s.* |
| Water | to 1 mL |

(c)

| | |
|---|---|
| Compound D | 10 mg |
| Acetic acid | 1.34 mg |
| Mannitol | 10 mg |
| Sodium hydroxide to pH 5 | *q.s*. |
| Water | to 1 mL |

(d)

| | |
|---|---|
| Compound D | 10 mg |
| Citric acid | 4.3 mg |
| Mannitol | 10 mg |
| Sodium hydroxide to pH 6 | *q.s*. |
| Water | to 1 mL |

(e)

| | |
|---|---|
| Compound D, methanesulphonic acid salt | 10 mg |
| Mannitol | 10 mg |
| Water | to 1 mL |

Mannitol is added prior to or after salt formation. The solutions are optionally sterile filtered, for example through a 0.22 µm membrane filter. Solutions (sterile or otherwise) are filled into appropriate vessels (e.g. vials) and the formulations are freeze-dried using standard equipment. Vials may be sealed in the freeze-dryer equipment under a nitrogen atmosphere.

Aqueous formulations, and freeze-dried compositions, comprising Compounds B and C, and salts thereof, are prepared in accordance with techniques described in the Examples above.

### Examples 12 to 16

### Immediate Release Tablets

### Tablet Manufacture

Tablets were manufactured using a standard tabletting machine (Kilian SP300) in accordance with standard procedures. Where appropriate, mixtures of drug and other excipients, were dry mixed (for example in a mortar) or wet or dry granulated using standard techniques.

### Test Method

Disintegration times for the tablets were determined using a *United States Pharmacopoeia XXIV* disintegration test method (as described on page 1941). Disintegration time is measured as the time within which the tablet is fully disintegrated in water.

### Example 12

96.3 mg of dibasic calcium phosphate, 7.7 mg of sodium starch glycolate, 7.7 mg of HPMC 6 cps and 3.8 mg of sodium stearyl fumarate were dry mixed together along with 250 mg of Compound D (free base). Tablets (punch diameter 10 mm) were compressed with a single punch press (Kilian SP300). The final tablet weight was about 365 mg. Disintegration time was 90 seconds in water (37°C).

### Example 13

100 mg of microcrystalline cellulose PH 102 SCG (special course grade; FMC International), 100 mg of microcrystalline cellulose PH 101 (FMC International), 30 mg of sodium starch glycolate, 10 mg of polyvinyl pyrrolidone K90 (approximate molcular weight 1,000,000; BASF) and 10 mg of sodium stearyl fumarate were dry mixed together along with 250 mg of Compound D (free base). Tablets (punch diameter 10 mm) were compressed with a single punch press (Kilian SP300). The final tablet weight was about 500 mg. Disintegration time was 6 minutes, 15 seconds in water (37°C).

### Example 14

43.75 mg of microcrystalline cellulose PH 102 (FMC International), 15.63 mg of polyvinyl pyrrolidone (crosslinked) and 3.13 mg of sodium stearyl fumarate were dry mixed together along with 250 mg of Compound D (free base). Tablets (punch diameter 10 mm) were compressed with a single punch press (Kilian SP300). The final tablet weight was about 312 mg. Disintegration time was less than 20 seconds in water (37°C).

### Example 15

28.13 mg of dibasic calcium phosphate, 15.63 mg of microcrystalline cellulose PH 102, 15.63 mg of polyvinyl pyrrolidone (crosslinked) and 3.13 mg of sodium stearyl fumarate were dry mixed together with 250 mg of Compound D (free base). Tablets (punch diameter 10 mm) were compressed with a single punch press (Kilian SP300). The final tablet weight was about 312 mg. Disintegration time was less then 20 seconds in water (37°C).

### Example 16

54.9 mg of microcrystalline cellulose PH 102 SCG, 30.5 mg of croscarmellose sodium (crosslinked sodium carboxymethylcellulose; FMC International), 30.5 mg of polyvinyl pyrrolidone K30 (approximate molcular weight 50,000; BASF) and 6.1 mg of sodium stearyl fumarate were dry mixed together with 500 mg of Compound D (free base). Tablets (punch diameter 12 mm) were compressed with a single punch press (Kilian SP300). The final tablet weight was about 610 mg. Disintegration time was 60 seconds in water (37°C).

### Abbreviations

- API =: atmospheric pressure ionisation (in relation to MS)
- br =: broad (in relation to NMR)
- d =: doublet (in relation to NMR)
- DCM =: dichloromethane
- DMF =: *N,N*-dimethylformamide
- DMSO =: dimethylsulfoxide
- dd =: doublet of doublets (in relation to NMR)
- Et =: ethyl
- eq. =: equivalents
- GC =: gas chromatography
- h =: hour(s)
- HCl =: hydrochloric acid
- HPLC =: high performance liquid chromatography
- IMS =: industrial methylated spirit
- IPA =: *iso*-propyl alcohol
- KF =: Karl-Fischer
- m =: multiplet (in relation to NMR)
- Me =: methyl
- MeCN =: acetonitrile
- min. =: minute(s)
- m.p. =: melting point
- MS =: mass spectroscopy
- Pd/C =: palladium on carbon
- q =: quartet (in relation to NMR)
- rt =: room temperature
- s =: singlet (in relation to NMR)
- t =: triplet (in relation to NMR)
- TLC =: thin layer chromatography
- UV =: ultraviolet

Prefixes *n-, s-, i-, t-* and *tert-* have their usual meanings: normal, secondary, *iso*, and tertiary.

## Claims

1. An immediate-release pharmaceutical formulation comprising, as active ingredient, 4({3-[7-(3,3-dimethyl-2-oxobutyl)-9-oxa-3,7-diazabi-cyclo[3.3.1]non-3-yl]propyl}amino)benzonitrile, *tert-*butyl 2-(7-[3-(4-cyanoanilino)propyl]-9-oxa-3,7-diazabicyclo[3.3.1]non-3-yl)ethyl-carbamate, *tert*-butyl 2-(7-[4-(4-cyanophenyl)butyl]-9-oxa-3,7-diazabi-cyclo[(3.3.1]non-3-yl}ethylcarbamate or *tert*-butyl 2-{7-((2*S*)-3-(4-cyanophenoxy)-2-hydroxypropyl](-9-oxa-3,7-diazabicyclo[3.3.1]non-3-yl}-ethylcarbamate or a pharmaceutically-acceptabte salt of any of these compounds: and a pharmaceutically-acceptable diluent or carrier.

2. A formulation as claimed in Claim 1, which releases at least 70% of the active ingredient within 4 hours of administration.

3. A formulation as claimed in Claim 2, wherein at least 80% of active ingredient is released.

4. A formulation as claimed in Claim 2 or Claim 3, wherein the release is within 1 hour.

5. A formulation as claimed in Claim 4, wherein the release is within 30 minutes.

6. A peroral immediate-release pharmaceutical formulation comprising, as active ingredient, 4-({3-[7-(3,3-dimeihy-2-oxobutyl)-9-oxa-3,7-diazabi-cyclo[3.3.1]non-3-yl]propyl}amino)benzonitrile, *tert*-butyl 2-{1-[3-(4-cyanoanilino)propyl]-9-oxa-3,7-diazabicyclo[3.3.1]non-3-yl}ethyl-carbamate, *tert*-butyl 2-{7-[4-(4-cyanophenyl)butyl]-9-oxa-3,7-diazabi-cyclo[3.3.1]non-3-yl}ethylcarbamate or *tert*-butyl 2-{7-[(2S)-3-(4-cyanophenoxy)-2-hydroxypropyl]-9-oxa-3,7-diazabicyclo[3.3.1]non-3-yl}-ethylcarbamate or a pharmaceutically-acceptable salt of any of these compounds; and a pharmaceutically-acceptable diluent or carrier, and which is in the form of a tablet, a capsule or a liquid dosage form.

7. A formulation as claimed in Claim 6, which is in the form of an immediate release tablet comprising active ingredient, diluent or carrier, and, optionally, one or more additional excipients.

8. A formulation as claimed in Claim 7, wherein the diluent or carrier is monobasic calcium phosphate, dibasic calcium phosphate (dihydrate or anhydrate), tribasic calcium phosphate, lactose, microcrystalline cellulose, silicified microcrystalline cellulose, mannitol, sorbitol, maize starch, potato starch, rice starch, glucose, calcium lactate or calcium carbonate.

9. A formulation as claimed in Claim 8, wherein the diluent or carrier is dibasic calcium phosphate (dihydrate or anhydrate) or microcrystalline cellulose.

10. A formulation as claimed in any one of Claims 7 to 9, wherein the optional additional excipient(s) comprise(s) a lubricant, a glidant, a binder and/or a disintegrant.

11. A formulation as claimed in Claim 10, wherein the lubricant is magnesium stearate, stearic acid, calcium stearate, stearyl alcohol or sodium stearyl fumarate

12. A formulation as claimed in Claim 11, wherein the lubricant is magnesium stearate or sodium stearyl fumarate.

13. A formulation as claimed in any one of Claims 10 to 12, wherein the lubricant is talc or a colloidal silica.

14. A formulation as claimed in any one of Claims 10 to 13, wherein the binder is polyvinylpyrrolidone, microcrystalline cellulose, a polyethylene glycol, a polyethylene oxide, a hydroxypropylmethylcellulose of a low molecular weight, a methylcellulose of a low molecular weight, a hydroxypropylcellulose of a low molecular weight, a hydroxyethylcellulose of a low molecular weight, maize starch, potato starch, rice starch or a sodium carboxymethyl cellulose of a low molecular weight

15. A formulation as claimed in Claim 14, wherein the binder is polyvinylpyrrolidone or a hydroxypropylmethylcellulose of a low molecular weight.

16. A formulation a claimed in any one of Claims 10 to 15, wherein the disintegrant is sodium starch glycolate, crosslinked polyvinylpyrrolidone, crosslinked sodium carboxymethyl cellulose, maize starch, potato starch, rice starch or an alginate.

17. A formulation a claimed in Claim 16, wherein the disintegrant is sodium starch glycolate, crosslinked polyvinylpyrrolidone or crosslinked sodium carboxymethyl cellulose.

18. A formulation as claimed in any one of Claims 7 to 17, wherein the amount of diluent/carrier in the formulation is up to 40% (wlw) of the final formulation.

19. A formulation as claimed in Claim 18, wherein the amount of diluent/carrier is up to 30% (w/w).

20. A formulation as claimed in Claim 19, wherein the amount of diluent/carrier is up to 20% (w/w).

21. A formulation as claimed in Claim 20, wherein the amount of diluent/carrier is up to 10% (w/w).

22. A formulation as claimed in any one of Claims 7 to 21, wherein the amount of additional excipient(s) is up to 5% (w/w) of the final formulation when the excipient(s) is/are a lubricant and/or a glidant.

23. A formulation as claimed in any one of Claims 7 to 21, wherein the amount of additional excipient(s) is up to 10% (w/w) of the final formulation when the excipient(s) is/are a binder and/or a disintegrant.

24. A parenteral immediate-release pharmaceutical formulation comprising, as active ingredient, 4-({3-[7-(3,3-dimethyl-2-oxobutyl)-9-oxa-3,7-diazabicyclo[3.3.1]non-3-yl]propyl}amino)benzonitrile, *tert*-butyl 2-{7-[3-(4-cyanoanilino)propyl]-9-oxa-3,7-diazabicyclo[3.3.1]non-3-yl}ethyl-carbamate, *tert*-butyl 2-{7-[4-(4-cyanophenyl)butyl]-9-oxa-3,7-diazabicyclo[3.3.1]non-3-yl)ethylcarbamate or *tert*-butyl 2-{7-[(2*S*)-3-(4-cyanophenoxy)-2-hydroxypropyl]-9-oxa-3,7-diazabicyclo[3.3.1]non-3-yl}-ethylcarbamate or a pharmaceutically-acceptable salt of any of these compounds; and a pharmaceutically-acceptable aqueous carrier, one or more additional excipients selected from the group antimicrobial preservatives, tonicity modifiers, pH adjusting agents, pH controlling agents, surfactants, cosolvents and/or antioxidants.

25. A formulation as claimed in Claim 24, wherein the administration is subcutaneous, intravenous, intraartetial, transdermal, intranasal, intrabuccal, intracutaneous, intramuscular, intralipomateous, intraperitoneal, rectal, sublingual, topical or by inhalation.

26. A formulation as claimed in Claim 24. wherein the tonicity modifier is selected from sodium chloride mannitol or glucose.

27. A formulation as claimed in Claim 24 or Claim 26, wherein the pH adjusting agent is hydrochloric acid or sodium hydroxide.

28. A formulation as claimed in any one of Claims 24 to 27, wherein the pH controlling agent is tartaric acid, acetic acid or citric acid.

29. A formulation as claimed in any one of Claims 24 to 28, wherein the cosolvent is ethanol, a polyethylene glycol or hydroxypropyl-β-cyclodextrin.

30. A formulation as claimed in any one of the preceding claims, in which the active ingredient is provided in the form of a methanesulphonic acid, a tartaric acid, a succinic acid, a citric acid, an acetic acid, a hippuric acid, a hydrochloric acid, or a hydrobromic acid, salt.

31. A formulation as claimed in any one of the preceding claims in which the active ingredient is 4-({3-[7-(3,3-dimethyl-2-oxobutyl)-9-oxa-3,7-diazabicyclo[3.3.1]non-3-yl]propyl}amino)benzonitrile or *tert*-butyl 2-(7-[(2*S*)-3-(4-cyanophenoxy)-2-hydroxypropyl]-9-oxa-3,7-diazabicyclo[3.3.1]-non-3-yl}ethylcarbamate, or a pharmaceutically acceptable salt of either compound.

32. A formulation as claimed in Claim 31, wherein the active ingredient is 4-({3-[7-(3,3-dimethyl-2-oxabutyl)-9-oxa-3,7-diazabicyclo[3.3.1]non-3-yl]propyl)amino)benzonitrile or a pharmaceutically acceptable salt thereof.

33. A formulation as claimed in Claim 32 in which, when the active ingredient is a salt of 4-({3-[7-(3,3-dimethyl-2-oxobutyl)-9-oxa-3,7-diazabicyclo[3.3.1]non-3-yl]propyl}amino)benzonitrile, it is a 1-hydroxy-2-naphthoic acid, a benzoic acid, a hydroxybenzenesulphonic acid, a benzenesulphonic acid, a toluenesulphonic acid, a naphthalenesulphonic acid, a naphthalenedisulphonic acid, a mesitylenesulphonic acid, a methanesulphonic acid, a tartaric acid, a succinic acid, a citric acid, an acetic acid, a hippuric acid, a hydrochloric acid, or a hydrobromic acid, salt.

34. A formulation as claimed in Claim 31, wherein the active ingredient is *tert*-butyl 2-{7-[(2*S*)-3-(4-cyanophenoxy)-2-hydroxypropyl]-9-oxa-3,7-diazabicyclo[3.3.1]-non-3-yl}ethylcarbamate or a pharmaceutically acceptable salt thereof.

35. A formulation as claimed in Claim 34, wherein the active ingredient is *tert*-butyl 2-{7-[(2*S*)-3-(4-cyanophenoxy)-2-hydroxypropyl]-9-oxa-3,7-diazabicyclo[3.3.1]non-3-yl}ethylcarbamate.

36. A formulation as claimed in Claim 34 in which, when the active ingredient is a salt of *tert*-butyl 2-{7-[(2*S*)-3-(4-cyanophenoxy)-2-hydroxypropyl]-9-oxa-3,7-diazabicyclo[3.3.1]-non-3-yl}ethylcarbamate, it is a L-lysine monohydrochloride, a pamoic acid, a terephthalic acid, a methanesulphonic acid, a tartaric acid, a succinic acid, a citric acid, an acetic acid, a hippuric acid, a benzoic acid, a hydrochloric acid, or a hydrobromic acid, salt.

37. A formulation as claimed in Claim 36 in which the salt is a methanesulphonic acid, a tartaric acid, a citric acid, or an acetic acid, salt.

38. A formulation as claimed in Claim 37 in which the salt is a methanesulphonic acid salt.

39. A formulation as claimed in any one of Claims 1 to 5 or 24 to 33 wherein, when the formulation of the invention comprises 4-({3-[7-(3,3-dimethyl-2-oxobutyl)-9-oxa-3,7-diazabicyclo[3.3.1]non3-yl]propyl}-amino)benzonitrile, either as the free base, as the *para*-toluenesulphonic acid salt, or as the benzenesulphonic add salt, and an aqueous carrier, along with ethanol as sole additional excipient, then the ethanol content is no more than 10% (w/w) of the content of the carrier.

40. A formulation as claimed in any one of Claims 1 to 5 or 24 to 39 which is an aqueous solution.

41. A formulation as claimed in any one of the preceding claims in which the active ingredient is water soluble.

42. A formulation as claimed in Claim 41, in which the solubility of active ingredient in aqueous solutions is at least 1 mg/mL.

43. A formulation as claimed in Claim 42, in which the solubility is at least 2 mg/mL.

44. A process for the preparation of a formulation as defined in any one of the preceding claims, which process comprises bringing active ingredient into association with a pharmaceutically-acceptable diluent or carrier.

45. A process as claimed in Claim 44 for the formation of a formulation as claimed in any one of Claims 6 to 24 which further comprises wet or dry granulation, and/or direct compression/compaction of active ingredient and diluent/carrier.

46. A process as claimed in Claim 44 for the formation of a formulation as claimed in any one of Claims 24 to 43 wherein, when active ingredient is in the form of an acid addition salt, the process further comprises addition of acid to 4-({3-[7-(3,3-dimethyl-2-oxobutyl)-9-oxa-3,7-diazabicyclo-[3.3.1]non-3-yl]propyl}amino)benzonitrile, *tert*-butyl 2-{7-[3-(4-cyanoanilino)propyl]-9-oxa-3,7-diazabicyclo[3.3.1]non-3-yl}ethylcarbamate, *tert*-butyl 2-{7-[4-(4-cyanophenyl)butyl]-9-oxa-3,7-diazabicyclo-[3.3.1]non-3-yl}ethylcarbamate or *tert*-butyl 2-{7-[(2S)-3-(4-cyanophenoxy)-2-hydroxypropyl]-9-oxa-3,7-diazabicyclo[3.3.1]non-3-yl}ethylcarbamate.

47. A process as claimed in Claim 46, wherein one or both of the acid or the base is provided in association with a diluent or carrier.

48. A process as claimed in Claim 46, wherein diluent or carrier is added to a mixture of acid and base.

49. A formulation as claimed in any one of Claims 1 to 5 or 24 to 43 which is suitable for direct administration to a patient.

50. A formulation as defined in any one of Claims 1 to 5 or 24 to 43 which is provided in the form of a concentrate of active ingredient and diluent or carrier, which concentrate is suitable for preparation of a formulation as claimed in Claim 49 by way of addition of further diluent or carrier prior to administration.

51. A process for the preparation of a formulation as defined in Claim 50, which comprises a process as defined in any one of Claims 44 or 46 to 48 followed by, if appropriate, concentration of the resultant formulation by removal of diluent or carrier.

52. A process as claimed in Claim 51, wherein the process of removal of diluent or carrier comprises evaporation (under reduced pressure or otherwise).

53. A process for the preparation of a formulation as defined in Claim 49 which comprises addition of diluent or carrier to a formulation as defined in Claim 50.

54. A solid pharmaceutical composition suitable for use in the preparation of a formulation as claimed in any one of Claims 1 to 5, 24 to 43.49 or 50 *ex tempore,* which composition comprises an active ingredient as defined in Claim 1.

55. A composition as claimed in Claim 54, which comprises active ingredient, one or more optional further excipients as defined in any one of Claims 24 to 29 and/or, optionally, up to 10% (w/w) of pharmaceutically-acceptable diluent or carrier.

56. A composition as claimed in Claim 54 or Claim 55, wherein, when the active ingredient is in the form of 4-({3-[7-(3,3-dimethyl-2-oxobutyl)-9-oxa-3,7-diazabicyclo[3.3.1]non-3-yl]propyl}amino)benzonitrile, 4-({3-[7-(3,3-dimethyl-2-oxobutyl)-9-oxa-3,7-diazabicyclo[3.3.1]non-3-yl]propyl}-amino)benzonitrile, benzenesulphonic acid salt, *tert*-butyl 2-[7-[3-(4-cyanoanilino)propyl]-9-oxa-3,7-diazabicyclo[3.3.1]non-3-yl}ethyl-carbamate, *tert*-butyl 2-{7-[4(4-cyanophenyl)butyl]-9-oxa-3,7-diazabicyclo[3.3.1]non-3 yl}ethylcarbamate, or *tert*-butyl 2-{7-[(2*S*)-3-(4-cyanophenoxy)-2-hydroxypropyl]-9-oxa-3,7-diazabicyclo[3.3.1]non-3-yl}-ethylcarbamate, then the composition comprises either one or more further excipients, or up to 10% (w/w) diluent or carrier.

57. A composition as claimed in any one of Claims 54 to 56. which composition is suitable for preparation of a pharmaceutically-acceptable solution *ex tempore.*

58. A composition as claimed in Claim 57, wherein the solution is an aqueous solution.

59. A composition as claimed in any one of Claims 54 to 58, wherein a further excipient is present which aids the formation of the solid composition during a process of removal of diluent or carrier.

60. A composition as claimed in Claim 59, wherein the further excipient is mannitol.

61. A process for the formation of a composition as claimed in any one of Claims 54 to 60 which comprises removal of diluent or carrier from a formulation as defined in any one of Claims 1 to 5, 24 to 43, 49 or 50.

62. A process as claimed in Claim 61, wherein the diluent or carrier is removed by evaporation (under reduced pressure or otherwise), spray drying or freeze-drying.

63. A process as claimed in Claim 62, wherein the diluent or carrier is removed by freeze drying.

64. A composition obtainable by a process according to any one of Claims 61 to 63.

65. A freeze-dried composition as defined in any one of Claims 54 to 60.

66. A formulation as defined in any one of Claims 1 to 43, 49 or 50, or a composition as defined in any one of Claims 54 to 60, 64 or 65, for use in medicine.

67. A formulation as defined in any one of Claims 1 to 43, 49 or 50, or a composition as defined in any one of Claims 54 to 60, 64 or 65, for use in the prophylaxis or the treatment of an arrhythmia.

68. The use of a formulation as defined in any of one Claims 1 to 43, 49 or 50, or a composition as defined in any one of Claims 54 to 60, 64 or 65, for the manufacture of a medicament for use in the prophylaxis or the treatment of an arrhythmia.

69. The use as claimed in Claim 68, wherein the arrhythmia is an atrial or a ventricular arrhythmia.

70. The use as claimed in Claim 68, wherein the arrhythmia is atrial fibrillation.

71. The use as claimed in Claim 68, wherein the arrhythmia is atrial flutter.

## Patentansprüche

1. Pharmazeutische Formulierung mit sofortiger Freisetzung, umfassend als wirksamen Inhaltsstoff 4-({3-[7-(3,3-Dimethyl-2-oxobutyl)-9-oxa-3,7-diazabi-cyclo[3.3.1]non-3-yl]propyl}amino)benzonitril, tert.-Butyl-2-{7-[3-(4-cyanoanilino)propyl]-9-oxa-3,7-diazabicyclo[3.3.1]non-3-yl}ethylcarbamat, tert.-Butyl-2-{7-[4-(4-cyanophenyl)butyl]-9-oxa-3,7-diazabi-cyclo[3.3.1]non-3-yl}ethylcarbamat oder tert.-Butyl-2-{7-[(2S)-3-(4-cyanophenoxy)-2-hydroxypropyl]-9-oxa-3,7-diazabicyclo[3.3.1]non-3-yl}-ethylcarbamat oder ein pharmazeutisch annehmbares Salz einer dieser Verbindungen; sowie ein pharmazeutisch annehmbares Verdünnungs- oder Trägermittel.

2. Formulierung nach Anspruch 1, die mindestens 70% des wirksamen Inhaltsstoffs innerhalb von vier Stunden nach Verabreichung freisetzt.

3. Formulierung nach Anspruch 2, wobei mindestens 80% des wirksamen Inhaltsstoffs freigesetzt wird.

4. Formulierung nach Anspruch 2 oder Anspruch 3, wobei die Freisetzung innerhalb von einer Stunde stattfindet.

5. Formulierung nach Anspruch 4, wobei die Freisetzung innerhalb von 30 Minuten stattfindet.

6. Perorale pharmazeutische Formulierung mit sofortiger Freisetzung, umfassend als wirksamen Inhaltsstoff 4-({3-[7-(3,3-Dimethyl-2-oxobutyl)-9-oxa-3,7-diazabicyclo[3.3.1]non-3-yl]propyl}amino)-benzonitril, tert.-Butyl-2-{7-[3-(4-cyanoanilino)-propyl]-9-oxa-3,7-diazabicyclo[3.3.1]non-3-yl}-ethyl-carbamat, tert.-Butyl-2-{7-[4-(4-cyanophenyl)butyl]-9-oxa-3,7-diazabicyclo[3.3.1]non-3-yl}ethylcarbamat oder tert.-Butyl-2-{7-[(2S)-3-(4-cyanophenoxy)-2-hydroxypropyl]-9-oxa-3,7-diazabicyclo[3.3.1]non-3-yl}-ethylcarbamat oder ein pharmazeutisch annehmbares Salz einer dieser Verbindungen; sowie ein pharmazeutisch annehmbares Verdünnungs- oder Trägermittel, wobei die Formulierung in Form einer Tablette oder einer Kapsel oder einer flüssigen Dosierungsform vorliegt.

7. Formulierung nach Anspruch 6, die in Form einer Tablette mit sofortiger Freisetzung, umfassend einen wirksamen Inhaltsstoff, ein Verdünnungs- oder Trägermittel und gegebenenfalls ein oder mehrere zusätzliche Hilfsstoffe, vorliegt.

8. Formulierung nach Anspruch 7, wobei es sich bei dem Verdünnungs- oder Trägermittel um monobasisches Calciumphosphat, dibasisches Calciumphosphat (Dihydrat oder Anhydrat), tribasisches Calciumphosphat, Lactose, mikrokristalline Cellulose, verkieselte mikrokristalline Cellulose, Mannit, Sorbit, Maisstärke, Kartoffelstärke, Reisstärke, Glucose, Calciumlactat oder Calciumcarbonat handelt.

9. Formulierung nach Anspruch 8, wobei es sich bei dem Verdünnungs- oder Trägermittel um dibasisches Calciumphosphat (Dihydrat oder Anhydrat) oder mikrokristalline Cellulose handelt.

10. Formulierung nach einem der Ansprüche 7 bis 9, wobei der optionale zusätzliche Hilfsstoff bzw. die optionalen zusätzlichen Hilfsstoffe ein Schmiermittel, ein Gleitmittel, ein Bindemittel und/oder ein Sprengmittel umfaßt bzw. umfassen.

11. Formulierung nach Anspruch 10, wobei sich bei dem Schmiermittel um Magnesiumstearat, Stearinsäure, Calciumstearat, Stearylalkohol oder Natriumstearylfumarat handelt.

12. Formulierung nach Anspruch 11, wobei sich bei dem Schmiermittel um Magnesiumstearat oder Natriumstearylfumarat handelt.

13. Formulierung nach einem der Ansprüche 10 bis 12, wobei es sich bei dem Schmiermittel um Talkum oder ein kolloidales Siliciumdioxid handelt.

14. Formulierung nach einem der Ansprüche 10 bis 13, wobei es sich bei dem Bindemittel um Polyvinylpyrrolidon, mikrokristalline Cellulose, ein Polyethylenglycol, ein Polyethylenoxid, eine Hydroxypropylmethylcellulose mit niedrigem Molekulargewicht, eine Methylcellulose mit niedrigem Molekulargewicht, eine Hydroxypropylcellulose mit niedrigem Molekulargewicht, eine Hydroxyethylcellulose mit niedrigem Molekulargewicht, Maisstärke, Kartoffelstärke, Reisstärke oder eine Natriumcarboxymethylcellulose mit niedrigem Molekulargewicht handelt.

15. Formulierung nach Anspruch 14, wobei es sich bei dem Bindemittel um Polyvinylpyrrolidon oder eine Hydroxypropylmethylcellulose mit niedrigem Molekulargewicht handelt.

16. Formulierung nach einem der Ansprüche 10 bis 15, wobei es sich bei dem Sprengmittel um Natriumstärkeglycolat, quervernetztes Polyvinylpyrrolidon, quervernetzte Natriumcarboxymethylcellulose, Maisstärke, Kartoffelstärke, Reisstärke oder ein Alginat handelt.

17. Formulierung nach Anspruch 16, wobei es sich bei dem Sprengmittel um Natriumstärkeglycolat, quervernetztes Polyvinylpyrrolidon oder quervernetzte Natriumcarboxymethylcellulose handelt.

18. Formulierung nach einem der Ansprüche 7 bis 17, wobei die Menge an Verdünnungs-/Trägermittel in der Formulierung bis zu 40 Gew.-% der endgültigen Formulierung beträgt.

19. Formulierung nach Anspruch 18, wobei die Menge an Verdünnungs-/Trägermittel bis zu 30 Gew.-% beträgt.

20. Formulierung nach Anspruch 19, wobei die Menge an Verdünnungs-/Trägermittel bis zu 20 Gew.-% beträgt.

21. Formulierung nach Anspruch 20, wobei die Menge an Verdünnungs-/Trägermittel bis zu 10 Gew.-% beträgt.

22. Formulierung nach einem der Ansprüche 7 bis 21, wobei die Menge an zusätzlichem Hilfsmittel bzw. zusätzlichen Hilfsmitteln bis zu 5 Gew.-% der endgültigen Formulierung beträgt, wenn es sich bei dem Hilfsmittel bzw. den Hilfsmitteln um ein Schmiermittel und/oder ein Gleitmittel handelt.

23. Formulierung nach einem der Ansprüche 7 bis 21, wobei die Menge an zusätzlichem Hilfsmittel bzw. zusätzlichen Hilfsmitteln bis zu 10 Gew.-% der endgültigen Formulierung beträgt, wenn es sich bei dem Hilfsmittel bzw. den Hilfsmitteln um ein Bindemittel und/oder ein Sprengmittel handelt.

24. Parenterale pharmazeutische Formulierung mit sofortiger Freisetzung, umfassend als wirksamen Inhaltsstoff 4-({3-[7-(3,3-Dimethyl-2-oxobutyl)-9-oxa-3,7-diazabi-cyclo[3.3.1]non-3-yl]propyl}amino)-benzonitril, tert.-Butyl-2-{7-[3-(4-cyanoanilino)-propyl]-9-oxa-3,7-diazabicyclo[3.3.1]non-3-yl}-ethylcarbamat, tert.-Butyl-2-{7-[4-(4-cyanophenyl)-butyl]-9-oxa-3,7-diazabi-cyclo[3.3.1]non-3-yl}-ethylcarbamat oder tert.-Butyl-2-{7-[(2S)-3-(4-cyanophenoxy)-2-hydroxypropyl]-9-oxa-3,7-diazabicyclo[3.3.1]non-3-yl}-ethylcarbamat oder ein pharmazeutisch annehmbares Salz einer dieser Verbindungen; sowie ein pharmazeutisch annehmbares wäßriges Trägermittel, ein oder mehrere zusätzliche Hilfsmittel, ausgewählt aus der Gruppe antimikrobielle Konservierungsstoffe, Mittel zum Einstellen der Tonizität, Mittel zum Einstellen des pH-Werts, Mittel zur Kontrolle des pH-Werts, Tenside, Cosolventien und/oder Antioxidationsmittel.

25. Formulierung nach Anspruch 24, wobei die Verabreichung subkutan, intravenös, intraarteriell, transdermal, intranasal, intrabukkal, intrakutan, intramuskulär, "intralipomatous" (in das Fettgewebe), intraperitonal, rektal, sublingual, topikal oder durch Inhalation erfolgt.

26. Formulierung nach Anspruch 24, wobei das Mittel zum Einstellen der Tonizität ausgewählt ist aus Natriumchlorid, Mannit oder Glucose.

27. Formulierung nach Anspruch 24 oder Anspruch 26, wobei es sich bei dem Mittel zum Einstellen des pH-Werts um Salzsäure oder Natriumhydroxid handelt.

28. Formulierung nach einem der Ansprüche 24 bis 27, wobei es sich bei dem Mittel zur Kontrolle des pH-Werts um Weinsäure, Essigsäure oder Zitronensäure handelt.

29. Formulierung nach einem der Ansprüche 24 bis 28, wobei es sich bei dem Cosolvenz um Ethanol, ein Polyethylenglykol oder um Hydroxypropyl-β-cyclodextrin handelt.

30. Formulierung nach einem der vorhergehenden Ansprüche, wobei der wirksame Inhaltsstoff in Form eines Salzes der Methansulfonsäure, der Weinsäure, der Bernsteinsäure, der Zitronensäure, der Essigsäure, der Hippursäure, der Salzsäure oder der Bromwasserstoffsäure handelt.

31. Formulierung nach einem der vorhergehenden Ansprüche, bei der es sich bei dem wirksamen Inhaltsstoff um 4-({3-[7-(3,3-Dimethyl-2-oxobutyl) -9-oxa-3,7-diazabicyclo[3.3.1]non-3-yl]propyl} amino)benzonitril oder tert.-Butyl-2-{7-[(2S)-3-(4-cyanophenoxy)-2-hydroxypropyl]-9-oxa-3,7-diazabicyclo[3.3.1]non-3-yl}ethylcarbamat oder jeweils ein pharmazeutisch annehmbares Salz der beiden Verbindungen handelt.

32. Formulierung nach Anspruch 31, wobei es sich bei dem wirksamen Inhaltsstoff um 4-({3-[7-(3,3-Dimethyl-2-oxobutyl)-9-oxa-3,7-diazabicyclo-[3.3.1]non-3-yl]propyl}amino)benzonitril oder ein pharmazeutisch annehmbares Salz davon handelt.

33. Formulierung nach Anspruch 32, bei der es sich bei dem wirksamen Inhaltsstoff, wenn es sich dabei um ein Salz von 4-({3-[7-(3,3-Dimethyl-2-oxobutyl)-9-oxa-3,7-diazabicyclo[3.3.1]non-3-yl]propyl}-amino)benzonitril handelt, um ein Salz der 1-Hydroxy-2-Naphthoesäure, der Benzoesäure, der Hydroxybenzolsulfonsäure, der Benzolsulfonsäure, der Toluolsulfonsäure, der Naphthalinsulphonsäure, der Naphthalindisulfonsäure, der Mesitylensulfonsäure, der Methansulfonsäure, der Weinsäure, der Bernsteinsäure, der Zitronensäure, der Essigsäure, der Hippursäure, der Benzoesäure, der Salzsäure oder der Bromwasserstoffsäure handelt.

34. Formulierung nach Anspruch 31, wobei es sich bei dem wirksamen Inhaltsstoff um tert.-Butyl-2-{7-[(2S)-3-(4-cyanophenoxy)-2-hydroxypropyl]-9-oxa-3,7-diazabicyclo[3.3.1]non-3-yl}ethylcarbamat oder ein pharmazeutisch annehmbares Salz davon handelt.

35. Formulierung nach Anspruch 34, wobei es sich bei dem wirksamen Inhaltsstoff um tert.-Butyl-2-{7-[(2S)-3-(4-cyanophenoxy)-2-hydroxypropyl]-9-oxa-3,7-diazabicyclo[3.3.1]non-3-yl}ethylcarbamat handelt.

36. Formulierung nach Anspruch 34, bei der es sich bei dem wirksamen Inhaltsstoff, wenn dieser ein Salz von tert.-Butyl-2-{7-[(2S)-3-(4-cyanophenoxy)-2-hydroxypropyl]-9-oxa-3,7-diazabicyclo[3.3.1]non-3-yl}ethylcarbamat ist, um ein L-Lysin-monohydrochlorid oder ein Salz der Pamoasäure der Terephthalsäure, der Methansulfonsäure, der Weinsäure, der Bernsteinsäure, der Zitronensäure, der Essigsäure, der Hippursäure, der Benzoesäure, der Salzsäure oder der Bromwasserstoffsäure handelt.

37. Formulierung nach Anspruch 36, bei der es sich bei dem Salz um ein Salz der Methansulfonsäure, der Weinsäure, der Zitronensäure oder der Essigsäure handelt.

38. Formulierung nach Anspruch 37, bei der es sich bei dem Salz um ein Salz der Methansulfonsäure handelt.

39. Formulierung nach einem der Ansprüche 1 bis 5 oder 24 bis 33, wobei, wenn die erfindungsgemäße Formulierung unter dem chemischen Namen 4-({3-[7-(3,3-Dimethyl-2-oxobutyl)-9-oxa-3,7-diazabicyclo-[3.3.1]non-3-yl]propyl}amino)benzonitril entweder als freie Base, als das para-Toluolsulfonsäuresalz oder als das Benzolsulfonsäuresalz sowie ein wäßriges Trägermittel zusammen mit Ethanol als einzigem zusätzlichem Hilfsmittel umfaßt, der Ethanolgehalt nicht mehr als 10 Gew.-% des Trägermittelgehalts beträgt.

40. Formulierung nach einem der Ansprüche 1 bis 5 oder 24 bis 39, bei der es sich um eine wäßrige Lösung handelt.

41. Formulierung nach einem der vorhergehenden Ansprüche, bei der der wirksame Inhaltsstoff wasserlöslich ist.

42. Formulierung nach Anspruch 41, wobei die Löslichkeit des wirksamen Inhaltsstoffs in wäßrigen Lösungen mindestens 1 mg/ml beträgt

43. Formulierung nach Anspruch 42, wobei die Löslichkeit mindestens 2 mg/ml beträgt.

44. Verfahren zur Herstellung einer Formulierung mit der in einem der vorhergehenden Ansprüche angegebenen Bedeutung, bei dem man den wirksamen Inhaltsstoff mit einem pharmazeutisch annehmbaren Verdünnungs- oder Trägermittel in Assoziation bringt.

45. Verfahren nach Anspruch 44 zur Bildung einer Formulierung nach einem der Ansprüche 6 bis 24, das ferner Feucht- oder Trockengranulation und/oder direktes Verpressen/ Kompaktieren des wirksamen Inhaltsstoffes und Verdünnungs-/Trägermittels umfaßt.

46. Verfahren nach Anspruch 44 zur Bildung einer Formulierung nach einem der Ansprüche 24 bis 43, wobei das Verfahren, wenn der wirksame Inhaltsstoff in Form eines Säureadditionssalzes vorliegt, ferner die Addition von Säure an 4-({3-[7-(3,3-Dimethyl-2-oxobutyl)-9-oxa-3,7-diazabicyclo[3.3.1]non-3-yl]propyl}amino)benzonitril, tert.-Butyl-2-{7-[3-(4-cyanoanilino)propyl]-9-oxa-3,7-diazabicyclo[3.3.1]non-3-yl}ethyl-carbamat, tert.-Butyl-2-{7-[4-(4-cyanophenyl)butyl]-9-oxa-3,7-diazabicyclo[3.3.1]non-3-yl}ethylcarbamat oder tert.-Butyl-2-{7-[(2S)-3-(4-cyanophenoxy)-2-hydroxy-propyl]-9-oxa-3,7-diazabicyclo[3.3.1]non-3-yl}-ethylcarbamat umfaßt.

47. Verfahren nach Anspruch 46, wobei die Säure und/oder die Base in Assoziation mit einem Verdünnungs- oder Trägermittel bereitgestellt wird.

48. Verfahren nach Anspruch 46, wobei das Verdünnungs- oder Trägermittel zu einem Gemisch aus Säure und Base gegeben wird.

49. Formulierung nach einem der Ansprüche 1 bis 5 oder 24 bis 43, die sich zur direkten Verabreichung an einen Patienten eignet.

50. Formulierung mit der in einem der Ansprüche 1 bis 5 oder 24 bis 43 angegebenen Bedeutung, die in Form eines Konzentrats von wirksamem Inhaltsstoff und Verdünnungs- oder Trägermittel bereitgestellt wird, wobei sich das Konzentrat zur Herstellung einer Formulierung nach Anspruch 49 über die Zugabe von weiterem Verdünnungs- oder Trägermittel vor der Verabreichung eignet.

51. Verfahren zur Herstellung einer Formulierung mit der in Anspruch 50 angegeben Bedeutung, das ein Verfahren mit der in einem der Ansprüche 44 oder 46 bis 48 angegeben Bedeutung und gegebenenfalls anschließend die Konzentration der erhaltenen Formulierung durch Abtrennen des Verdünnungs- oder Trägermittels umfaßt.

52. Verfahren nach Anspruch 51, wobei das Abtrennen des Verdünnungs- oder Trägermittels ein Eindampfen (unter vermindertem Druck oder sonstwie) umfaßt.

53. Verfahren zur Herstellung einer Formulierung mit der Anspruch 49 angegeben Bedeutung, das die Zugabe von Verdünnungs- oder Trägermittel zu einer Formulierung mit der in Anspruch 50 angegebenen Bedeutung umfaßt.

54. Feste pharmazeutische Zusammensetzung mit Eignung zur Verwendung bei der Herstellung einer Formulierung nach einem der Ansprüche 1 bis 5, 24 bis 43, 49 oder 50 *ex tempore,* wobei die Zusammensetzung einen wirksamen Inhaltsstoff mit der in Anspruch 1 angegebenen Bedeutung umfaßt.

55. Zusammmensetzung nach Anspruch 54, die einen wirksamen Inhaltsstoff, ein oder mehrere optionale weitere Hilfsmittel mit der in einem der Ansprüche 24 bis 29 angegebenen Bedeutung und/oder gegebenenfalls bis zu 10 Gew.-% eines pharmazeutisch annehmbaren Verdünnungs- oder Trägermittels umfaßt.

56. Zusammmensetzung nach Anspruch 54 oder Anspruch 55, wobei die Zusammensetzung, wenn der wirksame Inhaltsstoff in Form von 4-({3-[7-(3,3-Dimethyl-2-oxobutyl)-9-oxa-3,7-diazabicyclo[3.3.1]non-3-yl]propyl}amino)benzonitril, 4-({3-[7-(3,3-Dimethyl-2-oxobutyl)-9-oxa-3,7-diazabicyclo[3.3.1]-non-3-yl]propyl}-amino)benzonitril, Benzolsulfonsäuresalz, tert.-Butyl-2-{7-[3-(4-cyanoanilino)propyl]-9-oxa-3,7-diazabicyclo[3.3.1]non-3-yl)ethylcarbamat, tert.-Butyl-2-{7-[4-(4-cyanophenyl) butyl]-9-oxa-3,7-diazabicyclo[3.3.1]non-3-yl} ethylcarbamat oder tert.-Butyl-2-{7-[(2S)-3-(4-cyanophenoxy)-2-hydroxypropyl]-9-oxa-3,7-diazabicyclo[3.3.1]non-3-yl}-ethylcarbamat vorliegt, dann entweder ein oder mehrere weitere Hilfsmittel oder bis zu 10 Gew.-% Verdünnungs- oder Trägermittel umfaßt.

57. Zusammmensetzung nach einem der Ansprüche 54 bis 56, die sich zur Herstellung einer pharmazeutisch annehmbaren Lösung *ex tempore* eignet.

58. Zusammmensetzung nach Anspruch 57, wobei es sich bei der Lösung um eine wäßrige Lösung handelt.

59. Zusammmensetzung nach einem der Ansprüche 54 bis 58, wobei ein weiteres Hilfsmittel vorhanden ist, das die Bildung der festen Zusammensetzung während eines Abtrennens von Verdünnungs- oder Trägermitteln unterstützt.

60. Zusammmensetzung nach Anspruch 59, wobei es sich bei den weiteren Hilfsmitteln um Mannit handelt.

61. Verfahren zur Bildung einer Zusammensetzung nach einem der Ansprüche 54 bis 60, bei dem man Verdünnungs- oder Trägermittel aus einer Formulierung mit der in einem der Ansprüche 1 bis 5, 24 bis 43, 49 oder 50 angegebenen Bedeutungen entfernt.

62. Verfahren nach Anspruch 61, wobei das Verdünnungs- oder Trägermittel durch Eindampfen (unter vermindertem Druck oder sonstwie), Sprühtrocknen oder Gefriertrocknen abgetrennt wird.

63. Verfahren nach Anspruch 62, wobei das Verdünnungs- oder Trägermittel durch Gefriertrocknen abgetrennt wird.

64. Zusammmensetzung, erhältlich mit einem Verfahren nach einem der Ansprüche 61 bis 63.

65. Gefriergetrocknete Zusammensetzung mit der in einem der Ansprüche 54 bis 60 angegebenen Bedeutung.

66. Formulierung mit der in einem der Ansprüche 1 bis 43, 49 oder 50 angegebenen Bedeutung oder Zusammensetzung mit der in einem der Ansprüche 54 bis 60, 64 oder 65 angegebenen Bedeutung zur Verwendung in der Medizin.

67. Formulierung mit der in einem der Ansprüche 1 bis 43, 49 oder 50 angegebenen Bedeutung oder Zusammensetzung mit der in einem der Ansprüche 54 bis 60, 64 oder 65 angegebenen Bedeutung zur Verwendung bei der Prophylaxe oder Behandlung einer Arrhythmie.

68. Verwendung einer Formulierung mit der in einem der Ansprüche 1 bis 43, 49 oder 50 angegebenen Bedeutung oder einer Zusammensetzung mit der in einem der Ansprüche 54 bis 60, 64 oder 65 angegebenen Bedeutung zur Herstellung eines Arzneimittels zur Verwendung bei der Prophylaxe oder Behandlung einer Arrhythmie.

69. Verwendung nach Anspruch 68, wobei es sich bei der Arrhythmie um eine Vorhof-Arrhythmie oder eine ventrikuläre Arrhythmie handelt.

70. Verwendung nach Anspruch 68, wobei es sich bei der Arrhythmie um Vorhofflimmern handelt.

71. Verwendung nach Anspruch 68, wobei es sich bei der Arrhythmie um Vorhofflattern handelt.

## Revendications

1. Formulation pharmaceutique à libération immédiate, comprenant, comme ingrédient actif, du 4-({3-[7-(3,3-diméthyl-2-oxobutyl)-9-oxa-3,7-diazabicyclo[3.3.1]non-3-yl]propyl}amino)benzonitrile, du 2-{7-[3-(4-cyanoanilino)propyl]-9-oxa-3,7-diazabicyclo[3.3.1]non-3-yl}éthylcarbamate de tertio-butyle, du 2-{7-[4-(4-cyanophényl)butyl]-9-oxa-3,7-diazabicyclo[3.3.1]non-3-yl}éthylcarbamate de tertio-butyle ou du 2-{7-[(2S)-3-(4-cyanophénoxy)-2-hydroxypropyl]-9-oxa-3,7-diazabicyclo[3.3.1]non-3-yl}éthylcarbamate de tertio-butyle ou un sel pharmaceutiquement acceptable de l'un quelconque de ces composés ; et un diluant ou véhicule pharmaceutiquement acceptable.

2. Formulation selon la revendication 1, qui libère au moins 70% de l'ingrédient actif dans les 4 heures suivant l'administration.

3. Formulation selon la revendication 2, dans laquelle au moins 80% d'ingrédient actif est libéré.

4. Formulation selon la revendication 2 ou la revendication 3, dans laquelle la libération se produit au cours de la première heure.

5. Formulation selon la revendication 4, dans laquelle la libération se produit au cours des premières 30 minutes.

6. Formulation pharmaceutique perorale à libération immédiate, comprenant, comme ingrédient actif, du 4-({3-[7-(3,3-diméthyl-2-oxobutyl)-9-oxa-3,7-diazabicyclo[3.3.1]non-3-yl]propyl}amino)benzonitrile, du 2-{7-[3-(4-cyanoanilino)propyl]-9-oxa-3,7-diazabicyclo[3.3.1]non-3-yl}éthylcarbamate de tertio-butyle, du 2-{7-[4-(4-cyanophényl)butyl]-9-oxa-3,7-diazabicyclo[3.3.1]non-3-yl}éthylcarbamate de tertio-butyle ou du 2-{7-[(2S)-3-(4-cyanophénoxy)-2-hydroxypropyl]-9-oxa-3,7-diazabicyclo[3.3.1]non-3-yl}éthylcarbamate de tertio-butyle ou un sel pharmaceutiquement acceptable de l'un quelconque de ces composés ; et un diluant ou véhicule pharmaceutiquement acceptable, et qui se présente sous la forme d'un comprimé, d'une capsule ou sous une forme de dosage liquide.

7. Formulation selon la revendication 6, qui se présente sous la forme d'un comprimé à libération immédiate comprenant un ingrédient actif, un diluant ou véhicule, et éventuellement un ou plusieurs excipients supplémentaires.

8. Formulation selon la revendication 7, dans laquelle le diluant ou véhicule est le phosphate de calcium monobasique, le phosphate de calcium dibasique (dihydraté ou anhydraté), le phosphate de calcium tribasique, le lactose, la cellulose microcristalline, la cellulose microcristalline silicifiée, le mannitol, le sorbitol, l'amidon de maïs, l'amidon de pomme de terre, l'amidon de riz, le glucose, le lactate de calcium ou le carbonate de calcium.

9. Formulation selon la revendication 8, dans laquelle le diluant ou véhicule est le phosphate de calcium dibasique (dihydraté ou anhydraté) ou la cellulose microcristalline.

10. Formulation selon l'une quelconque des revendications 7 à 9, dans laquelle le ou les excipient(s) supplémentaire(s) facultatif(s) comprend (comprennent) un lubrifiant, un agent glissant, un liant et/ou un agent délitant.

11. Formulation selon la revendication 10, dans laquelle le lubrifiant est le stéarate de magnésium, l'acide stéarique, le stéarate de calcium, l'alcool stéarylique ou le stéarylfumarate de sodium.

12. Formulation selon la revendication 11, dans laquelle le lubrifiant est le stéarate de magnésium ou le stéarylfumarate de sodium.

13. Formulation selon l'une quelconque des revendications 10 à 12, dans laquelle le lubrifiant est le talc ou une silice colloïdale.

14. Formulation selon l'une quelconque des revendications 10 à 13, dans laquelle le liant est la polyvinylpyrrolidone, la cellulose microcristalline, un polyéthylène glycol, un oxyde de polyéthylène, une hydroxypropylméthylcellulose de bas poids moléculaire, une méthylcellulose de bas poids moléculaire, une hydroxypropylcellulose de bas poids moléculaire, une hydroxyéthylcellulose de bas poids moléculaire, l'amidon de maïs, l'amidon de pomme de terre, l'amidon de riz ou une carboxyméthylcellulose de sodium de bas poids moléculaire.

15. Formulation selon la revendication 14, dans laquelle le liant est la polyvinylpyrrolidone ou une hydroxypropylméthylcellulose de bas poids moléculaire.

16. Formulation selon l'une quelconque des revendications 10 à 15, dans laquelle l'agent délitant est le glycolate d'amidon sodique, la polyvinylpyrrolidone réticulée, la carboxyméthylcellulose de sodium réticulée, l'amidon de maïs, l'amidon de pomme de terre, l'amidon de riz ou un alginate.

17. Formulation selon la revendication 16, dans laquelle l'agent délitant est le glycolate d'amidon sodique, la polyvinylpyrrolidone réticulée ou la carboxyméthylcellulose de sodium réticulée.

18. Formulation selon l'une quelconque des revendications 7 à 17, dans laquelle la quantité de diluant/véhicule dans la formulation constitue jusqu'à 40% (p/p) de la formulation finale.

19. Formulation selon la revendication 18, dans laquelle la quantité de diluant/véhicule constitue jusqu'à 30% (p/p).

20. Formulation selon la revendication 19, dans laquelle la quantité de diluant/véhicule constitue jusqu'à 20% (p/p).

21. Formulation selon la revendication 20, dans laquelle la quantité de diluant/véhicule constitue jusqu'à 10% (p/p).

22. Formulation selon l'une quelconque des revendications 7 à 21, dans laquelle la quantité d'excipient(s) supplémentaire(s) constitue jusqu'à 5% (p/p) de la formulation finale lorsque le ou les excipient(s) est (sont) un lubrifiant et/ou un agent glissant.

23. Formulation selon l'une quelconque des revendications 7 à 21, dans laquelle la quantité d'excipient(s) supplémentaire(s) constitue jusqu'à 10% (p/p) de la formulation finale lorsque le ou les excipient(s) est (sont) un liant et/ou un agent délitant.

24. Formulation pharmaceutique parentérale à libération immédiate, comprenant, comme ingrédient actif, du 4-({3-[7-(3,3-diméthyl-2-oxobutyl)-9-oxa-3,7-diazabicyclo[3.3.1]non-3-yl]propyl}amino)benzonitrile, du 2-{7-[3-(4-cyanoanilino)propyl]-9-oxa-3,7-diazabicyclo[3.3.1]non-3-yl}éthylcarbamate de tertio-butyle, du 2-{7-[4-(4-cyanophényl)butyl]-9-oxa-3,7-diazabicyclo[3.3.1]non-3-yl}éthylcarbamate de tertio-butyle ou du 2-{7-[(2S)-3-(4-cyanophénoxy)-2-hydroxypropyl]-9-oxa-3,7-diazabicyclo[3.3.1]non-3-yl}éthylcarbamate de tertio-butyle ou un sel pharmaceutiquement acceptable de l'un quelconque de ces composés ; et un véhicule aqueux pharmaceutiquement acceptable, un ou plusieurs excipients supplémentaires choisis parmi le groupe constitué par les conservateurs antimicrobiens, les agents modifiant la tonicité, les agents d'ajustement du pH, les agents de contrôle du pH, les tensioactifs, les co-solvants et/ou les antioxydants.

25. Formulation selon la revendication 24, dans laquelle l'administration se fait par voie sous-cutanée, intraveineuse, intra-artérielle, transdermique, intra-nasale, intra-buccale, intra-cutanée, intramusculaire, intra-lipomateuse, intra-péritonéale, rectale, sublinguale, topique ou par inhalation.

26. Formulation selon la revendication 24, dans laquelle l'agent modifiant la tonicité est choisi parmi le chlorure de sodium, le mannitol et le glucose.

27. Formulation selon la revendication 24 ou la revendication 26, dans laquelle l'agent d'ajustement du pH est l'acide chlorhydrique ou l'hydroxyde de sodium.

28. Formulation selon l'une quelconque des revendications 24 à 27, dans laquelle l'agent de contrôle du pH est l'acide tartrique, l'acide acétique ou l'acide citrique.

29. Formulation selon l'une quelconque des revendications 24 à 28, dans laquelle le co-solvant est l'éthanol, un polyéthylène glycol ou l'hydroxypropyl-β-cyclodextrine.

30. Formulation selon l'une quelconque des revendications précédentes, dans laquelle l'ingrédient actif est fourni sous la forme d'un sel de l'acide méthanesulfonique, de l'acide tartrique, de l'acide succinique, de l'acide citrique, de l'acide acétique, de l'acide hippurique, de l'acide chlorhydrique ou de l'acide bromhydrique.

31. Formulation selon l'une quelconque des revendications précédentes, dans laquelle l'ingrédient actif est le 4-({3-[7-(3,3-diméthyl-2-oxobutyl)-9-oxa-3,7-diazabicyclo[3.3.1]non-3-yl]propyl}amino)benzonitrile ou le 2-{7-[(2S)-3-(4-cyanophénoxy)-2-hydroxypropyl]-9-oxa-3,7-diazabicyclo[3.3.1]non-3-yl}éthylcarbamate de tertio-butyle, ou un sel pharmaceutiquement acceptable de l'un ou l'autre de ces composés.

32. Formulation selon la revendication 31, dans laquelle l'ingrédient actif est le 4-({3-[7-(3,3-diméthyl-2-oxobutyl)-9-oxa-3,7-diazabicyclo[3.3.1]non-3-yl]propyl}amino)benzonitrile ou un sel pharmaceutiquement acceptable de celui-ci.

33. Formulation selon la revendication 32, dans laquelle, lorsque l'ingrédient actif est un sel du 4-({3-[7-(3,3-diméthyl-2-oxobutyl)-9-oxa-3,7-diazabicyclo[3.3.1]non-3-yl]propyl}amino)benzonitrile, il s'agit d'un sel de l'acide 1-hydroxy-2-naphtoïque, de l'acide benzoïque, de l'acide hydroxybenzènesulfonique, de l'acide benzènesulfonique, de l'acide toluènesulfonique, de l'acide naphtalènesulfonique, de l'acide naphtalènedisulfonique, de l'acide mésitylènesulfonique, de l'acide méthanesulfonique, de l'acide tartrique, de l'acide succinique, de l'acide citrique, de l'acide acétique, de l'acide hippurique, de l'acide benzoïque, de l'acide chlorhydrique ou de l'acide bromhydrique.

34. Formulation selon la revendication 31, dans laquelle l'ingrédient actif est le 2-{7-[(2S)-3-(4-cyanophénoxy)-2-hydroxypropyl]-9-oxa-3,7-diazabicyclo-[3.3.1]non-3-yl}éthylcarbamate de tertio-butyle, ou un sel pharmaceutiquement acceptable de celui-ci.

35. Formulation selon la revendication 34, dans laquelle l'ingrédient actif est le 2-{7-[(2S)-3-(4-cyanophénoxy)-2-hydroxypropyl]-9-oxa-3,7-diazabicyclo-[3.3.1]non-3-yl}éthylcarbamate de tertio-butyle.

36. Formulation selon la revendication 34, dans laquelle, lorsque l'ingrédient actif est un sel du 2-{7-[(2S)-3-(4-cyanophénoxy)-2-hydroxypropyl]-9-oxa-3,7-diazabicyclo[3.3.1]non-3-yl}éthylcarbamate de tertio-butyle, il s'agit d'un sel du monochlorhydrate de L-lysine, de l'acide pamoïque, de l'acide téréphtalique, de l'acide méthanesulfonique, de l'acide tartrique, de l'acide succinique, de l'acide citrique, de l'acide acétique, de l'acide hippurique, de l'acide benzoïque, de l'acide chlorhydrique ou de l'acide bromhydrique.

37. Formulation selon la revendication 36, dans laquelle le sel est un sel de l'acide méthanesulfonique, de l'acide tartrique, de l'acide citrique ou de l'acide acétique.

38. Formulation selon la revendication 37, dans laquelle le sel est un sel de l'acide méthanesulfonique.

39. Formulation selon l'une quelconque des revendications 1 à 5 ou 24 à 33, dans laquelle, lorsque la formulation de l'invention comprend du 4-({3-[7-(3,3-diméthyl-2-oxobutyl)-9-oxa-3,7-diazabicyclo-[3.3.1]non-3-yl]propyl}amino)benzonitrile, soit comme base libre, comme sel de l'acide *para-*toluènesulfonique, ou bien comme sel de l'acide benzènesulfonique, et un véhicule aqueux, conjointement avec de l'éthanol comme seul excipient supplémentaire, alors la teneur en éthanol n'est pas supérieure à 10% (p/p) de la teneur en véhicule.

40. Formulation selon l'une quelconque des revendications 1 à 5 ou 24 à 39, qui est une solution aqueuse.

41. Formulation selon l'une quelconque des revendications précédentes, dans laquelle l'ingrédient actif est hydrosoluble.

42. Formulation selon la revendication 41, dans laquelle la solubilité de l'ingrédient actif en solutions aqueuses est d'au moins 1 mg/ml.

43. Formulation selon la revendication 42, dans laquelle la solubilité est d'au moins 2 mg/ml.

44. Procédé de préparation d'une formulation telle que définie selon l'une quelconque des revendications précédentes, lequel procédé comprend la mise en association de l'ingrédient actif avec un diluant ou véhicule pharmaceutiquement acceptable.

45. Procédé selon la revendication 44 de formation d'une formulation selon l'une quelconque des revendications 6 à 24, comprenant en outre la granulation par voie sèche ou humide, et/ou la compaction/compression directe de l'ingrédient actif et du diluant/véhicule.

46. Procédé selon la revendication 44 de formation d'une formulation selon l'une quelconque des revendications 24 à 43, dans lequel, lorsque l'ingrédient actif est sous la forme d'un sel d'addition d'acide, le procédé comprend en outre l'addition d'acide au 4-({3-[7-(3,3-diméthyl-2-oxobutyl)-9-oxa-3,7-diazabicyclo[3.3.1]non-3-yl]propyl}-amino)benzonitrile, au 2-{7-[3-(4-cyanoanilino)propyl]-9-oxa-3,7-diazabicyclo[3.3.1]non-3-yl}éthylcarbamate de tertio-butyle, au 2-{7-[4-(4-cyanophényl)butyl]-9-oxa-3,7-diazabicyclo[3.3.1]non-3-yl}éthylcarbamate de tertio-butyle ou au 2-{7-[(2S)-3-(4-cyanophénoxy)-2-hydroxypropyl]-9-oxa-3,7-diazabicyclo[3.3.1]non-3-yl}-éthylcarbamate de tertio-butyle.

47. Procédé selon la revendication 46, dans lequel un ou les deux parmi l'acide ou la base est fourni en association avec un diluant ou véhicule.

48. Procédé selon la revendication 46, dans lequel le diluant ou véhicule est ajouté à un mélange d'acide et de base.

49. Formulation selon l'une quelconque des revendications 1 à 5 ou 24 à 43, qui est convenable pour une administration directe à un patient.

50. Formulation telle que définie selon l'une quelconque des revendications 1 à 5 ou 24 à 43, qui est fournie sous la forme d'un concentrat d'ingrédient actif et de diluant ou véhicule, lequel concentrat est convenable pour la préparation d'une formulation selon la revendication 49 au moyen de l'addition de diluant ou véhicule supplémentaire préalablement à l'administration.

51. Procédé de préparation d'une formulation telle que définie selon la revendication 50, comprenant un procédé tel que défini selon l'une quelconque des revendications 44 ou 46 à 48, suivi, le cas échéant, de la concentration de la formulation résultante par l'élimination du diluant ou véhicule.

52. Procédé selon la revendication 51, dans lequel le procédé d'élimination du diluant ou véhicule comprend une évaporation (sous pression réduite ou autre).

53. Procédé de préparation d'une formulation telle que définie selon la revendication 49, comprenant l'addition de diluant ou véhicule à une formulation telle que définie selon la revendication 50.

54. Composition pharmaceutique solide convenable pour une utilisation dans la préparation d'une formulation selon l'une quelconque des revendications 1 à 5, 24 à 43, 49 ou 50, extemporanément, laquelle composition comprend un ingrédient actif tel que défini selon la revendication 1.

55. Composition selon la revendication 54, comprenant un ingrédient actif, un ou plusieurs excipients supplémentaires facultatifs tels que définis selon l'une quelconque des revendications 24 à 29 et/ou, éventuellement, jusqu'à 10% (p/p) d'un diluant ou véhicule pharmaceutiquement acceptable.

56. Composition selon la revendication 54 ou la revendication 55, dans laquelle, lorsque l'ingrédient actif est sous la forme de 4-({3-[7-(3,3-diméthyl-2-oxobutyl)-9-oxa-3,7-diazabicyclo[3.3.1]non-3-yl]propyl}amino)benzonitrile, de sel de l'acide benzènesulfonique du 4-({3-[7-(3,3-diméthyl-2-oxobutyl)-9-oxa-3,7-diazabicyclo[3.3.1]non-3-yl]propyl}amino)benzonitrile, de 2-{7-[3-(4-cyanoanilino)propyl]-9-oxa-3,7-diazabicyclo[3.3.1]non-3-yl}éthylcarbamate de tertio-butyle, de 2-{7-[4-(4-cyanophényl)butyl]-9-oxa-3,7-diazabicyclo[3.3.1]non-3-yl}éthylcarbamate de tertio-butyle ou de 2-{7-[(2S)-3-(4-cyanophénoxy)-2-hydroxypropyl]-9-oxa-3,7-diazabicyclo[3.3.1]non-3-yl}éthylcarbamate de tertio-butyle, alors la composition comprend soit un ou plusieurs excipients supplémentaires, soit jusqu'à 10% (p/p) de diluant ou véhicule.

57. Composition selon l'une quelconque des revendications 54 à 56, laquelle composition est convenable pour la préparation extemporanée d'une solution pharmaceutiquement acceptable.

58. Composition selon la revendication 57, dans laquelle la solution est une solution aqueuse.

59. Composition selon l'une quelconque des revendications 54 à 58, dans laquelle un excipient supplémentaire est présent, qui favorise la formation de la composition solide pendant un procédé d'élimination du diluant ou véhicule.

60. Composition selon la revendication 59, dans laquelle l'excipient supplémentaire est le mannitol.

61. Procédé de formation d'une composition selon l'une quelconque des revendications 54 à 60, comprenant l'élimination du diluant ou véhicule d'une formulation telle que définie selon l'une quelconque des revendications 1 à 5, 24 à 43, 49 ou 50.

62. Procédé selon la revendication 61, dans lequel le diluant ou véhicule est éliminé par évaporation (sous pression réduite ou autre), séchage par pulvérisation ou lyophilisation.

63. Procédé selon la revendication 62, dans lequel le diluant ou véhicule est éliminé par lyophilisation.

64. Composition pouvant être obtenue par un procédé selon l'une quelconque des revendications 61 à 63.

65. Composition lyophilisée telle que définie selon l'une quelconque des revendications 54 à 60.

66. Formulation telle que définie selon l'une quelconque des revendications 1 à 43, 49 ou 50, ou composition telle que définie selon l'une quelconque des revendications 54 à 60, 64 ou 65, pour une utilisation en médecine.

67. Formulation telle que définie selon l'une quelconque des revendications 1 à 43, 49 ou 50, ou composition telle que définie selon l'une quelconque des revendications 54 à 60, 64 ou 65, pour une utilisation dans la prophylaxie ou le traitement d'une arythmie.

68. Utilisation d'une formulation telle que définie selon l'une quelconque des revendications 1 à 43, 49 ou 50, ou d'une composition telle que définie selon l'une quelconque des revendications 54 à 60, 64 ou 65, pour la fabrication d'un médicament destiné à une utilisation dans la prophylaxie ou le traitement d'une arythmie.

69. Utilisation selon la revendication 68, dans laquelle l'arythmie est une arythmie auriculaire ou ventriculaire.

70. Utilisation selon la revendication 68, dans laquelle l'arythmie est une fibrillation auriculaire.

71. Utilisation selon la revendication 68, dans laquelle l'arythmie est un flutter auriculaire.
